# EUROPEAN PATENT APPLICATION

(11) **EP 3 733 848 A1**
(43) Date of publication of application: **04.11.2020**
(21) Application number: 20172761.7
(22) Date of filing: 14.12.2012
(51) Int. Cl.: C12N 15/11, A61K 9/16, A61K 48/00, A61K 31/713, A61K 47/54, A61K 47/55, A61K 47/60, A61P 31/00, A61P 35/00, A61P 43/00

(54) **ANTISENSE OLIGONUCLEOTIDE CONJUGATES AND USE THEREOF**

(30) Priority: 15.12.2011 KR 20110135162; 05.01.2012 KR 20120001710
(62) Divisional of application: 12857568.5
(71) Applicant: Bioneer Corporation, Daejeon 306-220 (KR)
(72) Inventor: CHAE, Jeiwook, 305-761 Daejeon (KR); HAN, Boram, 420-859 Bucheon-si Gyeonggi-do (KR); KIM, Han-na, 560-886 Jeonju-si Jeollabuk-do (KR); PARK, Han Oh, 305-761 Daejeon (KR); YOON, Pyoung Oh, 305-752 Daejeon (KR); KIM, Sun Gi, 305-509 Daejeon (KR); JUNG, Kwang-Ju, 612-052 Busan (KR); KWON, Taewoo, 305-753 Daejeon (KR); LEE, Sam Young, 305-758 Daejeon (KR); JUNG, Eun-Jung, 336-799 Asan-si Chungcheongnam-do (KR); CHOI, Jong Deok, 305-753 Daejeon (KR)
(74) Representative: Hiebl, Inge Elisabeth

(57) **Abstract**

The present invention provides a double-stranded RNA structure, which comprises a polymer compound covalently bonded to a double-helix oligo RNA useful for the treatment of diseases, particularly cancer, in order to enhance the delivery of the double-helix oligo RNA, and further comprises a target-specific ligand bonded thereto, a preparation method thereof, and a technique of delivering the double-helix oligo RNA in a target-specific manner using the RNA structure. A nanoparticle composed of the ligand-bonded double-helix oligo RNA structures can efficiently deliver the double-helix oligo RNA to a target, and thus can exhibit the activity of the double-helix oligo RNA even when the double-helix oligo RNA is administered at a relatively low concentration. Also, it can prevent the non-specific delivery of the double-helix oligo RNA into other organs and cells. Accordingly, the ligand-bonded double-stranded RNA structure can be used for the treatment for various diseases, particularly cancer, and can also be effectively used as a new type of double-helix oligo RNA delivery system. Particularly, the ligand-bonded double-stranded RNA structure can be effectively used for the treatment of diseases, including cancer and infectious diseases. Moreover, the present invention relates to a hybrid conjugate, which comprises a hydrophilic material and hydrophobic material bonded to both ends of an antisense oligonucleotide (ASO) by a covalent bond in order to enhance the in vivo stability of the ASO, a method for preparing the hybrid conjugate, and a nanoparticle composed of the conjugates. The ASO-polymer conjugate according to the invention can increase the *in vivo* stability of the ASO, making it possible to efficiently deliver the therapeutic ASO into cells. Also, the ASO-polymer conjugate can exhibit the activity of the ASO even when it is administered at a relatively low concentration.

## Description

### TECHNICAL FIELD

The present invention relates to a novel oligonucleotide structure having bonded thereto hydrophilic and hydrophobic materials that enhance the delivery of single-stranded or double-stranded oligonucleotides useful for the treatment of cancer and infectious diseases, and to the use thereof.

A first aspect of the present invention relates to a double-helix oligo RNA structure comprising a target-specific ligand bonded to a hydrophilic material contained in the structure, a nanoparticle composed of the ligand-bonded double-helix oligo RNA structures, a pharmaceutical composition comprising the RNA structure, a pharmaceutical composition comprising a nanoparticle composed of the ligand-bonded double-helix oligo RNA structures, a method for preparing the structure, a method for preparing a nanoparticle composed of the RNA structures, and a technique for delivery of the ligand-bonded double-helix oligo-RNA structure.

A second aspect of the present invention relates to an antisense oligonucleotide (hereinafter referred to as "ASO") conjugate which comprises a hydrophilic material and hydrophobic material bonded to both ends of the ASO by a simple covalent bond or a linker-mediated covalent bond in order to enhance the intracellular delivery efficiency of the ASO, a method for preparing the conjugate, and a technique for delivery of a nanoparticle composed of the ASO conjugates.

### BACKGROUND ART

Since the role of RNA interference (hereinafter referred to as 'RNAi') was found, it has been found that RNAi acts in a sequence specific mRNA on a variety of mammalian cells (Silence of the transcripts: RNA interference in medicine. J Mol Med (2005) 83: 764-773). When a long double-stranded RNA is delivered into cells, the delivered RNA is processed by the endonuclease dicer into 21-23 base pair (bp) small interfering RNA (hereinafter referred to as 'siRNA'). siRNA binds to RISC (RNA-induced silencing complex) and inhibits the expression of the target gene in a sequence-specific manner by the process in which the antisense strand recognizes and degrades the target mRNA (NUCLEIC-ACID THERAPEUTICS: BASIC PRINCIPLES AND RECENT APPLICATIONS. Nature Reviews Drug Discovery. 2002. 1, 503-514).

Bertrand et al. reported that siRNA has an excellent inhibitory effect on the expression of mRNA *in vitro* and *in vivo* compared to an antisense oligonucleotide (ASO) for the same target gene and that the effect is long lasting (Comparison of antisense oligonucleotides and siRNAs in cell culture and in vivo. Biochem. Biophys. Res. Commun.2002. 296: 1000-1004). Also, because siRNA complementarily binds to the target mRNA to regulate the expression of the target gene in a sequence-specific manner, it can be advantageously used in a wide range of applications compared to conventional antibody-based drugs or chemicals (small molecule drugs) (Progress Towards in Vivo Use of siRNAs. Molecular Therapy. 2006 13(4) :664-670).

siRNA has excellent effects and can be used in a wide range of applications, but it order for siRNA to be developed into cell therapeutic agents, it is required to improve the stability and intracellular delivery efficiency of siRNA so as to effectively deliver siRNA into its target cells (Harnessing in vivo siRNA delivery for drug discovery and therapeutic development. Drug Discov. Today. 2006 Jan; 11(1-2):67-73).

In an attempt to satisfy these requirements, nuclease-resistant analogues or carriers such as viral vectors, liposomes or nanoparticles have been used.

Viral carriers such as adenovirus or retrovirus have high transfection efficacy, but carry the risks of immunogenicity and oncogenicity. However, non-viral carriers including nanoparticles are evaluated to have low intracellular delivery efficiency compared to viral carriers, but have advantages, including high safety *in vivo,* target-specific delivery, efficient uptake and internalization of RNAi oligonucleotides into cells or tissues, and low cytotoxicity and immune stimulation. Thus, these non-viral carriers are considered to be the most promising delivery method that makes to effectively inhibit the expression of the target gene (Nonviral delivery of synthetic siRNAs in vivo. J. Clin Invest. 2007 December 3; 117(12): 3623-3632).

Delivery systems with various nanoparticles have been developed for cancer-specific delivery. Such nanoparticle systems are usually designed such that the surface is coated with a hydrophilic material to increase the time of circulation in blood and is positively charged to increase endocytosis (Active targeting schemes for nanoparticle systems in cancer therapeutics. Advanced Drug Delivery Reviews 60 (2008) 1615-1626). Meanwhile, tumor tissue is very rigid and has diffusion limitation, unlike normal tissue, and overcomes the diffusion limitation by forming blood vessels in the surrounding region by angiogenesis, because this diffusion limitation have adverse affects on the migration of nutrients required for tumor, and waste materials such as oxygen and carbon dioxide. The blood vessels formed in tumor tissue by angiogenesis have a leaky and defective blood vessel including a gap having a size ranging from about 100 nm to 2 µm depending on the kind of tumor.

Thus, nanoparticles easily pass through the capillary endothelium of cancer tissue having a leaky and defective blood vessel, compared to the structured capillary vessels of normal tissue, so that they are easily delivered during their circulation in blood. In addition, tumor tissue has no lymphatic drainage, and thus a drug is accumulated therein. This mechanism is known as the enhanced permeation and retention (EPR) effect. Nanoparticles are easily delivered specifically into tumor tissue by this effect, and this mechanism is known as passive targeting (Nanoparticles for drug delivery in cancer treatment. Urol Oncol. 2008 Jan-Feb; 26(1):57-64).

To overcome the non-specific in vivo distribution, targeting and lack of water solubility of therapeutic drugs including anticancer drugs, studies have been conducted to optimize the size of nanoparticles loaded with therapeutic drugs or modify the surface to increase the time of their circulation in blood. Particularly, with respect to polymeric nanoparticles comprising polymer-drug conjugates, studies have been conducted to enhance the tumor-specific delivery of anticancer drugs by linking the anticancer drugs to water-soluble, biodegradable materials such as albumin, poly-L-glutamate (PGA) or an N- (2-hydroxypropyl)-methacrylamide copolymer (Therapeutic Nanoparticles for Drug Delivery in Cancer. Clin Cancer Res 2008; 14: 1310-1316). In addition, studies have been conducted to link an amphiphilic material to an anticancer drug so as to form polymeric micelles consisting of a hydrophobic core of anticancer drug and a hydrophilic shell (Development of the polymer micelle carrier system for doxorubicin. J Control Release 2001; 74: 295-302).

Thus, when a hydrophobic material is additionally bound to a therapeutic drug such as an anticancer drug to increase the cohesive force of the core, micelles can be formed even at low concentration, and polymer micelles having increased stability due to the hydrophilic material of the shell can be formed. A therapeutic drug having hydrophobic and hydrophilic materials bound to both ends by a biodegradable bond can form improved polymer micelles that can stably deliver the therapeutic drug into the target cancer tissue.

Recently, as technology for delivering double-stranded oligo RNA, technology of the self-assembled nanoparticle SAMiRNA formed based on the characteristics of materials bound to the ends of nucleic acid was developed (Korean Patent Laid-Open Publication No. 2009-0042297). SAMiRNA is a self-assembled nanoparticle composed of double-stranded oligo RNA structures having bound thereto hydrophilic and hydrophobic materials that enhance the delivery of double-stranded oligo RNA, and technology for forming SAMiRNA can be technology for enhancing the intracellular delivery of double-stranded oligo RNA.

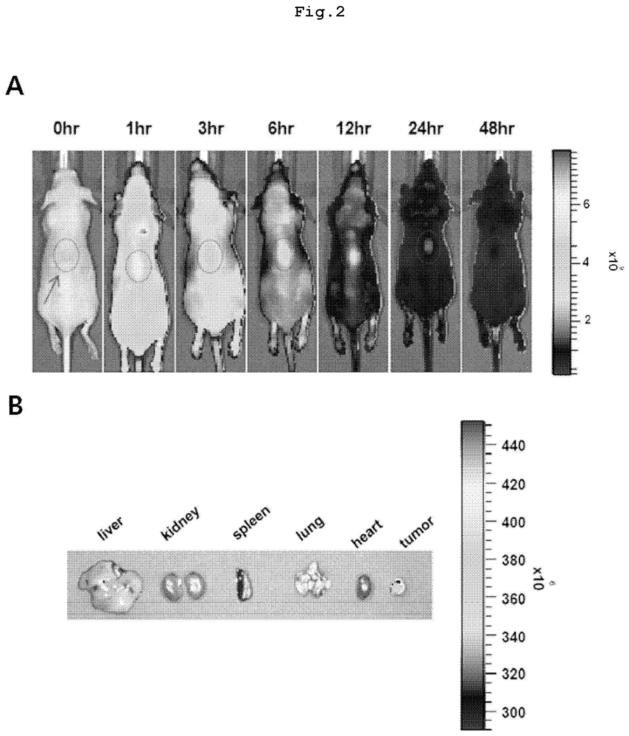

It was found that, when SAMiRNA labeled with a fluorescent tag was administered to the tail vein of a tumor xenograft mouse model, the nanoparticle was delivered specifically to tumor by the above-mentioned passive targeting (see FIG. 2).

Meanwhile, active targeting uses nanoparticles having a targeting moiety bound thereto. It was reported that the targeting moiety causes the preferential accumulation of nanoparticles in the target tissue or enhances the internalization of nanoparticles into the target cells (Does a targeting ligand influence nanoparticle tumor localization or uptake Trends Biotechnol. 2008 Oct; 26(10):552-8. Epub 2008 Aug 21).

Active targeting means enhancing the delivery of nanoparticles to the target cells using a targeting moiety such as an antibody or a ligand, bound to the nanoparticles. In recent years, studies have been conducted to localize siRNAs to a desired tissue using various targeting moieties bound to the siRNAs.

For example, it was found that an siRNA having α-tocopherol bound thereto was effectively and stably delivered in vivo and inhibited the expression of the target gene by RNA interference (Kazutaka Nishina et al., The American Society of Gene therapy, 2008, 16(4):734-740). Also, it was shown that an siRNA having cholesterol bound thereto was more effectively delivered into liver tissue compared to a cell penetrating-peptide (CCP) that is mainly used for the delivery of siRNA (US-20060014289; Moschos S.A. et al., Bioconjug. Chem. 18:1450-1459). It was reported that the delivering effect is caused not only by the specificity of tumor tissue, but also by the specificity of a cell targeted by the bound targeting moiety.

Active targeting uses materials having the capability to bind to carbohydrates, receptors or antigens, which are specific for or overexpressed on the target cell surface (Nanotechnology in cancer therapeutics: bioconjugated nanoparticles for drug delivery. Mol Cancer Ther 2006; 5(8) : 1909-1917). Thus, nanoparticles having an active targeting moiety bound thereto are accumulated in tumor tissue during their circulation in blood by passive targeting, and the delivery of the nanoparticles into the target cells is enhanced by the targeting moiety, thus increasing the therapeutic effect of the drug delivered into the cells. As the targeting moiety, a ligand or an antibody is mainly used. It binds to its receptor on the cell surface with high avidity and specificity and promotes the internalization of the nanoparticles by receptor-mediated endocytosis (RME) (Kinetic analysis of receptor-mediated endocytosis (RME) of proteins and peptides: use of RME as a drug delivery system. J Control Release 1996; 39: 191-200).

The cell surface receptor or antigen that is targeted by this ligand or antibody has a characteristic in that it is specific for or overexpressed in the target cells to facilitate the access of the targeting ligand thereto, thereby increasing the rate of endocytosis. In addition, the receptor or antigen delivers the nanoparticles having the ligand bound thereto into the cells, and is recycled back to the cell surface (Receptor-mediated endocytosis: An overview of a dynamic process. J. Biosci., Oct. 1984, 6(4), pp. 535-542.). Tumor targeting moieties are materials that bind specifically either to receptors such as epidermal growth factor or low-density lipoprotein receptor, which are expressed specifically expressed in the target cell lines, or to tumor-specific receptors such as folate receptor, which are known to be overexpressed on the surface of various cancer cells (Nanotechnology in cancer therapeutics: bioconjugated nanoparticles for drug delivery. Mol Cancer Ther 2006, 5(8): 1909-1917).

If a targeting moiety, particularly a receptor-specific ligand that enhances internalization by receptor-mediated endocytosis (RME), is bonded to SAMiRNA, it can efficiently promote the delivery of the SAMiRNA into the target cells, particularly cancer cells, and thus the SAMiRNA can be delivered into the target cells even at a relatively low concentration and dose so that the double-stranded oligo RNA can exhibit high activity and the non-specific delivery of the double-stranded oligo RNA into other organs and cells can be inhibited.

In addition, the SAMiRNA forming technology may be applied not only to double-stranded oligo RNAs, but also to single-stranded oligonucleotides, particularly a single-stranded antisense oligonucleotide (ASO) for therapeutic purposes.

ASO technology is the technology of controlling the transfer of information from gene to protein by changing the metabolism of mRNA using single-stranded RNA or DNA. In other words, it is the technology of performing the preferential inhibition of expression of the protein of interest using a selected nucleotide sequence that complementarily and specifically hybridizes to the protein. Because ASO binds to the target gene in a sequence-specific manner, it does not influence the expression of genes other than the target gene. Thus, the ASO technology can serve as a useful tool in the analysis of the *in vivo* function of a specific protein and can also be used as gene therapy against a specific disease (FASEBJ 9, 1288-1296, 1995).

In recent years, an antagomir that is a new type of single-stranded antisense oligonucleotide was developed and has been used to inhibit the function of microRNAs in cells. It is known that an antagomir or microRNA inhibitor (miRNA inhibitor) that is a chemically synthesized short RNA binds complementarily to the target microRNA to inhibit the function of the microRNA. An antagomir preferably has a modified chemical structure such as 2' methoxy or phosphothioate to prevent the degradation of the antagomir. Currently, antagomirs that inhibit the functions of miRNAs related to various diseases, including cancer and cardiac and pulmonary fibrosis, are known ("Silencing of microRNAs in vivo with 'antagomirs'" Nature, Dec. 2005, 438(7068): 685-689; "MicroRNAs as Therapeutic Targets" New England J. Medicine, 2006, 354 (11): 1194-1195; Meister G. et al., "Sequence-specific inhibition of microRNA- and siRNA-induced RNA silencing" RNA, Mar. 2004, 10 (3): 544-550).

Antisense DNA binds to the target mRNA to form a RNA/DNA duplex, which is degraded by RNase H (a kind of ribonuclease that specifically degrades an mRNA having a RNA/DNA hybrid duplex formed therein) *in vivo.* Antisense RNA forms a RNA/RNA duplex, and the degradation of the target mRNA is induced by RNase L. RNase L is a ribonuclease that preferentially degrades single-stranded RNA around double-stranded RNA (Pharmacol.Toxicol.32,329-376,1992).

However, an ASO comprising the miRNA inhibitor antagomir should be effectively delivered into the target cells to achieve a desired effect, and the ASO can be degraded by ribonuclease in blood. Thus, in order to use an ASO for therapeutic purposes, an ASO conjugate should be efficiently delivered through the cell membrane, and the stability of the ASO *in vivo* should be ensured (Shigeru Kawakami and Mitsuru Hashida, Drug Metab. Pharmacokinet. 22(3): 142-151, 2007).

Thus, in order to the *in vivo* stability, most ASOs are in the form of oligodeoxynucleotides (ODNs) obtained by various modifications that provide nuclease resistance. The modification can be the substitution of an -OH group at the 2' carbon position of the sugar moiety of one or more nucleotides with -CH₃ (methyl), -OCH₃, -NH₂, -F(fluorine), -O-2-methoxyethyl, -O-propyl, -O-2-methylthioethyl, -0-3-aminopropyl, -O-3-dimethylaminopropyl, -O-*N*-methylacetamido or -O-dimethylamidoxyethyl; the substitution of oxygen of the sugar moiety of the nucleotide with sulfur; modification of the bond between the nucleotides into a phosphorothioate, boranophosphophate or methyl phosphonate bond; or a combination of one or more thereof; or modification in the form of PNA (peptide nucleic acid) or LNA (locked nucleic acid) (see Crooke et al., Ann. Rev. Med. Vol.55: pp 61-65 2004, US 5,660,985, US 5,958,691, US 6,531,584, US 5,808,023, US 6,326,358, US 6,175,001 Braasch D. A. et al., Bioorg. Med. Chem. Lett. 14:1139-1143, 2003; Chiu Y. L. et al., RNA, 9:1034-1048, 2003; Amarzguioui M. et al., Nucleic Acid Res. 31:589-595, 2003).

In order to deliver ASOs into the target cells, gene delivery techniques that use viruses such as adenovirus or retrovirus, and gene delivery techniques that use non-viral carriers such as liposomes, cationic lipids or cationic polymers, have been developed. However, viral carriers have problems in terms of safety, because it is not guaranteed that these carriers do not cause abnormalities in the normal functions of host genes after incorporation into the host chromosome, or do not activate oncogenes. Also, if the viral gene is continuously expressed even at low levels to cause autoimmune diseases or if the viral carrier causes modified viral infection, ASOs cannot be efficiently delivered.

To overcome such problems, methods of fusing a gene to the non-viral carrier liposome or methods of using cationic lipids or polymers have been studied to overcome the shortcomings thereof. Although these non-viral carriers are less efficient than viral carriers, these have advantages in that they are safe *in vivo,* cause less side effects, and can be produced at low costs (Lehrman S. Nature. 401(6753):517-518, 1999).

In order to effectively achieve the stable delivery of ODN molecules including an ASO using non-viral carriers, an effective method of preventing enzymatic or non-enzymatic degradation is required. Thus, methods of chemically modifying ASOs to make the AOSs stable against nuclease and increase the intracellular absorption of the AOSs have been proposed (Shigery Kawakami and Mitsuru Hashida. Drug Metab.Parmacokinet. 22(3): 142-151, 2007).

Meanwhile, polymers comprising PEG (polyethylene glycol) form composites having micelle structures spontaneously formed by interactions therebetween, and these composites are known as polymer composite micelles (Kataoka K. et al. Macromolecules, 29:8556-8557, 1996). These polymer composite micelles have advantages in that they have a very small size compared to other drug delivery systems such as microspheres or nanoparticles while the distribution thereof is very uniform, and they are spontaneously formed, making it easy to control the quality of the formulation and ensure reproducibility.

In recent years, in order to increase the intracellular delivery efficiency of ASOs, the technology of ensuring the stability of ASOs and the efficient permeation of ASOs through the cell membrane by conjugating a hydrophilic material such as the biocompatible polymer PEG to ASOs by a simple covalent bond or a linker-mediated covalent bond was developed (Korean Patent Registration No. 0466254). However, improving the *in vivo* stability of ASOs and ensuring the efficient delivery of ASOs into the target tissue are difficult to achieve by only chemical modification and PEGylation.

As described above, the SAMiRNA technology about nanoparticles obtained by introducing hydrophobic and hydrophilic materials to siRNA to enhance the intracellular delivery of the siRNA was developed, but the application of this technology to the delivery of ASOs has not yet been reported. Thus, there is a need to develop an ASO delivery system and a preparation method thereof by introducing various chemical modifications into ASOs and conjugating various polymers to ASOs to protect the ASOs from enzymes to thereby increase the stability thereof and the efficient permeation thereof through the cell membrane.

### DISCLOSURE OF INVENTION

In a first aspect, an object of the present invention is to provide a therapeutic drug structure, which comprises hydrophilic and hydrophobic materials which are biocompatible polymer compounds bonded to both ends of the therapeutic drug by a simple covalent bond or a linker-mediated covalent bond in order to increase the intracellular delivery efficiency of the therapeutic drug, and further comprises a ligand bonded to the hydrophilic material, a nanoparticle composed of the therapeutic drug structure, and a preparation method thereof.

Another object of the present invention is to provide a double-helix oligo RNA structure, which comprises biocompatible hydrophilic and hydrophobic polymer materials bonded to both ends of the double-helix oligo RNA by a simple bond or a linker-mediated covalent bond in order to increase the intracellular delivery efficiency of the double-helix oligo RNA, and further comprises, bonded to the hydrophilic material, a receptor-specific ligand having the property of enhancing internalization of the target cell (particularly cancer cell) by receptor-mediated endocytosis (RME); a nanoparticle composed of the ligand-bonded double-helix oligo RNA structures; and a pharmaceutical composition comprising either the ligand-bonded double-helix oligo RNA structure or a nanoparticle composed of the ligand-bonded double-helix oligo RNA structures.

Still another object of the present invention is to provide methods for preparing the ligand-bonded double-helix oligo RNA structure and a nanoparticle comprising the same, and a technique of delivering a double-helix oligo RNA using the ligand-bonded double-helix oligo RNA structure.

When a target-specific ligand is bonded to a double-helix oligo RNA structure, a nanoparticle composed of the ligand-bonded double-helix oligo RNA structures can be efficiently delivered into the target cell. Thus, even when the ligand-bonded double-helix oligo RNA structure is administered at a relatively low concentration, it can exhibit the activity of the double-helix oligo RNA in the target cell. Further, because the bonded ligand can prevent the non-specific delivery of the double-helix oligo RNA into other organs and cells, the ligand-bonded double-helix RNA structure can be used for the treatment for various diseases and can also be effectively used as a new type of double-helix oligo RNA delivery system. Particularly, the ligand-bonded double-helix RNA structure can be effectively used for the treatment of diseases, including cancer and infectious diseases.

In a second aspect, an object of the present invention is to provide an ASO-polymer conjugate, which comprises biocompatible hydrophilic and hydrophobic polymer materials bonded to both ends of the ASO by a simple covalent bond or a linker-mediated covalent bond in order to enhance the intracellular delivery efficiency of the ASO, and a preparation method thereof.

Another object of the present invention is to provide a technique of delivering an ASO using a nanoparticle composed of the ASO-polymer conjugates, and a pharmaceutical composition comprising either the ASO-polymer conjugate or a nanoparticle composed of the ASO-polymer conjugates.

The ASO-polymer conjugate according to the present invention and a nanoparticle composed of the ASO-polymer conjugates can increase the *in vivo* stability of the ASO, making it possible to efficiently deliver the therapeutic ASO into cells. Also, they can exhibit the activity of the ASO at relatively low concentrations compared to an ASO whose end was not modified, even in the absence of a transfection agent. Thus, the ASO-polymer conjugate and a nanoparticle composed of the ASO-polymer conjugates can be used for the treatment of various diseases, including cancer and infectious diseases, and can also be very effectively used as a new type of ASO delivery system in basic bioengineering research and medical industries.

Moreover, the invention comprises the following embodiments:
1. A therapeutic drug-polymer structure having a structure of the following formula (1) and comprising a ligand bonded thereto:

   Formula 1 L-A-X-R-Y-B

   wherein A is a hydrophilic material; B is a hydrophobic material; X and Y are each a simple covalent bond or a linker-mediated covalent bond independently of each other; R is a therapeutic drug; and L is a receptor-specific ligand having the property of enhancing internalization of the target cell by receptor-mediated endocytosis (RME).
2. The therapeutic drug-polymer structure of item 1, wherein the therapeutic drug is a double-helix oligo RNA or an anticancer drug.
3. The therapeutic drug-polymer structure of item 1, wherein the ligand is selected from among target-specific antibodies, aptamers, peptides, and receptor-specific chemical materials, which specifically bind to a target and perform receptor-mediated endocytosis (RME).
4. The therapeutic drug-polymer structure of item 3, wherein the receptor-specific chemical materials are selected from among folate, N-acetylgalactosamine (NAG), and mannose.
5. The therapeutic drug-polymer structure of item 2, wherein the double-helix oligo RNA is composed of 19-31 nucleotides.
6. The therapeutic drug-polymer structure of item 2 wherein the double-helix oligo RNA comprises modification comprising the substitution of an -OH group at the 2' carbon position of the sugar moiety of one or more nucleotides with -CH₃ (methyl), -OCH₃, -NH₂, -F(fluorine), -O-2-methoxyethyl, - O-propyl, -O-2-methylthioethyl, -O-3-aminopropyl, -0-3-dimethylaminopropyl, -O-N-methylacetamido or -O-dimethylamidoxyethyl; the substitution of oxygen in the sugar moiety of the nucleotide with sulfur; modification of the bond between the nucleotides into one or a combination of two or more selected from the group consisting of a phosphorothioate, boranophosphophate and methyl phosphonate bond, or is modified in the form of PNA (peptide nucleic acid) or LNA (locked nucleic acid).
7. The therapeutic drug-polymer structure of item 1, wherein the hydrophobic material has a molecular weight of 250-1,000.
8. The therapeutic drug-polymer structure of item 7, wherein the hydrophobic material is selected from the group consisting of a steroid derivative, a glyceride derivative, glycerol ether, polypropylene glycol, a C₁₂-C₅₀ unsaturated or saturated hydrocarbon, diacyl phosphatidylcholine, fatty acid, phospholipid, and lipopolyamine.
9. The therapeutic drug-polymer structure of item 8, wherein the steroid derivative is selected from the group consisting of cholesterol, cholestanol, cholic acid, cholesteryl formate, cholestanyl formate, and cholestanyl amine.
10. The therapeutic drug-polymer structure of item 8 wherein the glyceride derivative is selected from among mono-, di- and tri-glycerides.
11. The therapeutic drug-polymer structure of item 1, wherein the hydrophilic material has a molecular weight of 200-10,000.
12. The therapeutic drug-polymer structure of item 11, wherein the hydrophilic material is selected from the group consisting of polyethylene glycol, polyvinyl pyrolidone, and polyoxazoline.
13. The therapeutic drug-polymer structure of item 1, wherein the covalent bond is either a non-degradable bond or a degradable bond.
14. The therapeutic drug-polymer structure of item 13, wherein the non-degradable bonds is either an amide bond or a phosphate bone.
15. The therapeutic drug-polymer structure of item 13, wherein the degradable bonds is selected from the group consisting of a disulfide bond, an acid-degradable bond, an ester bond, an anhydride bond, a biodegradable bond or an enzymatically degradable bond.
16. A method for preparing a ligand-conjugated double-helix oligo RNA structure, the method comprising the steps of:
   (1) synthesizing a single-stranded RNA on a solid support having a functional group-hydrophilic material bonded thereto;
   (2) covalently bonding a hydrophobic material to the 5' end of the single-stranded RNA having the functional group-hydrophilic material bonded thereto;
   (4) separating the functional group-RNA-polymer structure and a separately synthesized complementary single-stranded RNA from the solid support;
   (5) bonding a ligand to the end of the hydrophilic material by the functional group; and
   (6) annealing the ligand-bonded RNA-polymer structure with the complementary single-stranded RNA to form a double-stranded RNA structure.
17. A method for preparing a double-helix oligo RNA structure, the method comprising the steps of:
   (1) synthesizing a single-stranded RNA on a solid support;
   (2) covalently bonding a hydrophilic material to the 5' end of the single-stranded RNA;
   (3) bonding a ligand to the hydrophilic material bonded to the single-stranded RNA;
   (4) separating the ligand-bonded, RNA-hydrophilic polymer structure and a separately synthesized complementary RNA-hydrophobic polymer structure from the solid support; and
   (5) annealing the ligand-bonded, RNA-hydrophilic polymer structure with the complementary RNA-hydrophobic polymer structure to form a double-stranded structure,
   wherein the preparation method comprises, between steps (1) to (4), a step of synthesizing a single-stranded RNA complementary to the single-stranded RNA of step (1), and then covalently bonding a hydrophobic material to the synthesized single-stranded RNA to synthesize a single-stranded RNA-hydrophobic polymer structure.
18. A method for preparing a ligand-bonded double-helix oligo RNA structure, the method comprising the steps of:
   (1) synthesizing a single-stranded RNA on a solid support having a functional group bonded thereto;
   (2) covalently bonding a hydrophilic material to the material obtained in step (1);
   (3) covalently bonding a ligand to the material obtained in step (2);
   (4) separating the material obtained in step (3) from the solid support;
   (5) covalently bonding a hydrophobic material to the material resulting from step (4) by the functional group bonded to the 3' end; and
   (6) annealing the material resulting from step (5) with a complementary single-stranded RNA to form a double-strand RNA structure.
19. A nanoparticle comprising the therapeutic drug-polymer structure of any one of items 1 to 15.
20. A pharmaceutical composition comprising the therapeutic drug-polymer structure of any one of items 1 to 15.
21. A pharmaceutical composition comprising the nanoparticle of item 19.
22. An antisense oligonucleotide (ASO)-polymer conjugate represented by the following formula 5:

   Formula 5 A-X-R-Y-B

   wherein one of A and B is a hydrophilic material, the other one is a hydrophobic material, X and Y are each a simple covalent bond or a linker-mediated covalent bond independently of each other, and R is an ASO.
23. The antisense oligonucleotide (ASO)-polymer conjugate of item 22, wherein the ASO is composed of 10-50 oligonucleotides.
24. The antisense oligonucleotide (ASO)-polymer conjugate of item 23, wherein the oligonucleotides
   comprises modification comprising the substitution of an -OH group at the 2' carbon position of the sugar moiety of one or more nucleotides with -CH₃ (methyl), -OCH₃, -NH₂, -F (fluorine), -O-2-methoxyethyl, -O-propyl, -0-2-methylthioethyl, -O-3-aminopropyl, -O-3-dimethylaminopropyl, -O-N-methylacetamido or -O-dimethylamidoxyethyl; the substitution of oxygen in the sugar moiety of the nucleotide with sulfur; modification of the bond between the nucleotides into one or a combination of two or more selected from the group consisting of a phosphorothioate, boranophosphophate and methyl phosphonate bond, or is modified in the form of PNA (peptide nucleic acid) or LNA (locked nucleic acid).
25. The antisense oligonucleotide (ASO)-polymer conjugate of item 22, wherein the hydrophilic material has a molecular weight of 200-10,000.
26. The antisense oligonucleotide (ASO)-polymer conjugate of item 25, wherein the hydrophilic material is selected from the group consisting of polyethylene glycol, polyvinyl pyrolidone, and polyoxazoline.
27. The antisense oligonucleotide (ASO)-polymer conjugate of item 22, wherein the hydrophobic material has a molecular weight of 250-1,000.
28. The antisense oligonucleotide (ASO)-polymer conjugate of item 27, wherein the hydrophobic material is either a C₁₂-C₅₀ hydrocarbon or cholesterol.
29. The antisense oligonucleotide (ASO)-polymer conjugate of item 22, wherein the covalent bond is either a non-degradable bond or a degradable bond.
30. The antisense oligonucleotide (ASO)-polymer conjugate of item 29, wherein the non-degradable bonds is either an amide bond or a phosphate bone.
31. The antisense oligonucleotide (ASO)-polymer conjugate of item 29, wherein the degradable bonds is selected from the group consisting of a disulfide bond, an acid-degradable bond, an ester bond, an hydride bond, a biodegradable bond or an enzymatically degradable bond.
32. An ASO-polymer conjugate comprising a ligand bonded to a hydrophilic material of the ASO-polymer conjugate of any one of item 22 to 31.
33. A method for preparing an ASO-polymer conjugate, the method comprising the steps of:
   (a) covalently bonding a hydrophilic material to a solid support;
   (b) synthesizing an ASO on the solid support comprising the hydrophilic material;
   (c) covalently bonding a hydrophobic material to the 5' end of the ASO on the solid support; and
   (d) separating and purifying the resulting ASO-polymer conjugate from the solid support.
34. A method for preparing an ASO-polymer conjugate, the method comprising the steps of:
   (a) synthesizing an ASO on a solid support having a functional group bonded thereto;
   (b) covalently bonding a hydrophilic material to the 5' end of the ASO;
   (c) separating the hydrophilic material-bonded ASO conjugate from the solid support; and
   (d) covalently bonding a hydrophobic material to the 3' end of the ASO separated from the solid support.
35. A method for preparing an ASO-polymer conjugate comprising a ligand attached thereto, the method comprising the steps of:
   (a) bonding a hydrophilic material to a solid support having a functional group attached thereto;
   (b) synthesizing an ASO on the solid support having the functional group-hydrophilic material bonded thereto;
   (c) covalently bonding a hydrophobic material to the 5' end of the ASO;
   (d) separating an ASO-polymer conjugate, obtained in step (c), from the solid support; and
   (e) bonding a ligand to the hydrophilic material of the ASO-polymer conjugate separated from the solid support.
36. A method for preparing an ASO-polymer conjugate comprising a ligand attached thereto, the method comprising the steps of:
   (a) synthesizing an ASO on a solid support having a functional group attached thereto;
   (b) covalently bonding a hydrophilic material to the end of the ASO;
   (c) covalently bonding a ligand to the ASO-hydrophilic material conjugate;
   (d) separating an ASO-hydrophilic polymer-ligand conjugate, which has the functional group attached thereto, from the solid support; and
   (e) covalently bonding a hydrophobic material to the 3' end of the ASO of the conjugate separated from the solid conjugate.
37. A nanoparticle comprising the ASO-polymer conjugate of any one of items 22 to 31.
38. A nanoparticle comprising the ligand bonded ASO-polymer conjugate of item 32.
39. A pharmaceutical composition comprising a pharmaceutically effective amount of the ASO-polymer conjugate of any one of items 22 to 31.
40. A pharmaceutical composition comprising a pharmaceutically effective amount of the nanoparticle of item 37.
41. A pharmaceutical composition comprising a pharmaceutically effective amount of the ligand bonded ASO-polymer conjugate of items 32.
42. A pharmaceutical composition comprising a pharmaceutically effective amount of the nanoparticle of item 38.

### BRIEF DESCRIPTION OF THE DRAWINGS

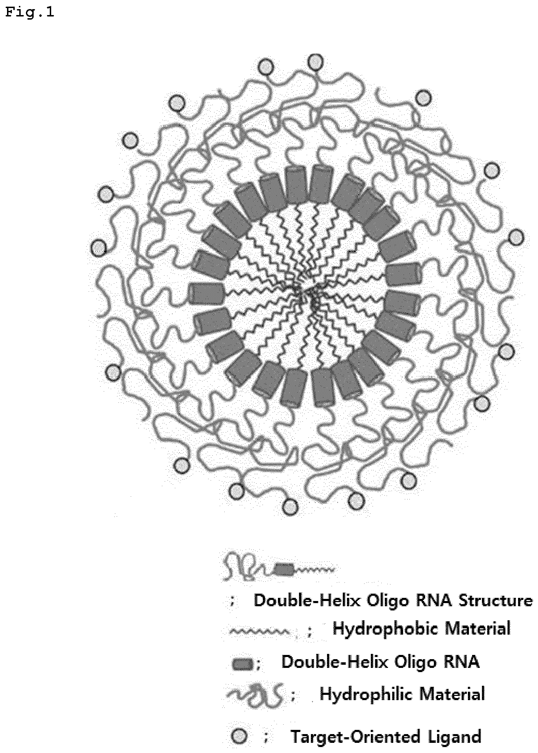

FIG. 1 is a schematic view of a nanoparticle (SAMiRNA) composed of double-helix oligo RNA structures having a ligand bonded thereto.
FIG. 2 shows the tumor-specific delivery of SAMiRNA. FIG. 2A is a photograph showing the biodistribution of SAMiRNA with time after Cy5.5-labeled SAMiRNA was administered once to the tail vein of a tumor-transplanted mouse at a dose 5 mg/kg body weight (the portion indicated by the red dotted line is a tumor-transplanted portion). FIG. 2(B) is an ex vivo photograph of each tissue collected at 48 hours after administration of SAMiRNA.

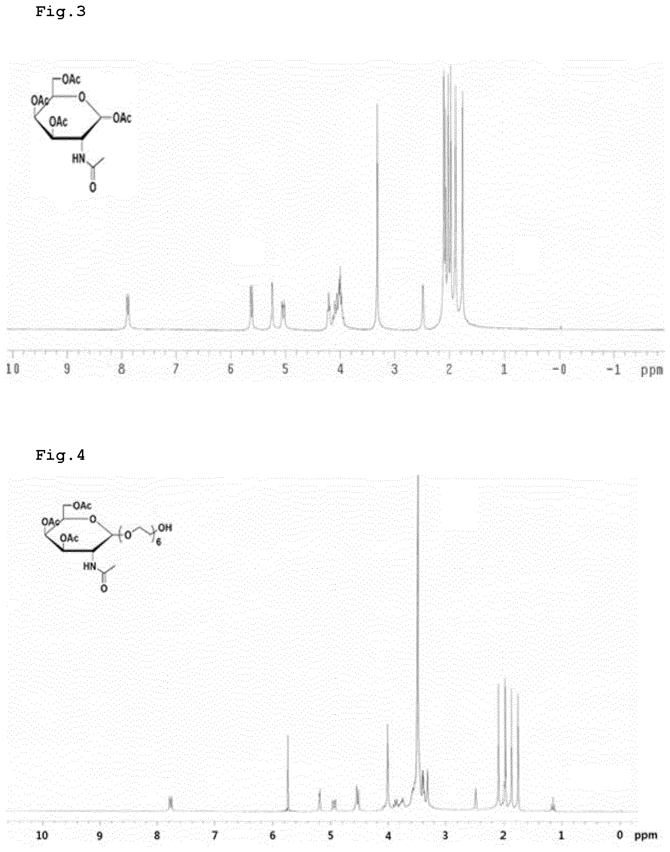

FIG. 3 shows the results of NMR analysis of 1,3,4,6-tetraacetyl-NAG (compound A). ¹H NMR (300 MHz, DMSO-D6); δ7.89 ppm (1H, d, J=9.3 Hz), 5.64 ppm (1H, d, J=8.7 Hz), 5.27 ppm (1H, d, J=3.3 Hz), 5.07ppm (1H, dd, J=11.7, 3.6 Hz), 4.22 ppm (1H, t, J=6.3 Hz), 4.14-3.96 ppm (2H, m), 2.12ppm (3H, s), 2.04 ppm (3H, s), 1.99 ppm (3H, s), 1.91(3H, s), 1.78 ppm (3H, s) .
FIG. 4 shows the results of NMR analysis of 3,4,6-triacetyl-1-hexa(ethylene glycol)-N-acetylgalactosamine(NAG) (compound B). ¹H NMR (300 MHz, DMSO-D₆); δ7.71 ppm (1H, d, J=9.3 Hz), 5.21 ppm (1H, d, J=3.0 Hz), 4.97 ppm (1H, dd, J=11.1, 3.0 Hz), 4.56 ppm (1H, d, J=8.7 Hz), 3.88 ppm (1H, q, J=8.7 Hz), 3.83-3.74ppm (1H, m), 3.62-3.39 ppm (25H, m), 2.10 ppm (3H, s), 2.01 ppm (3H, s), 1.89 ppm (3H, s), 1.77 ppm (3H, s) .

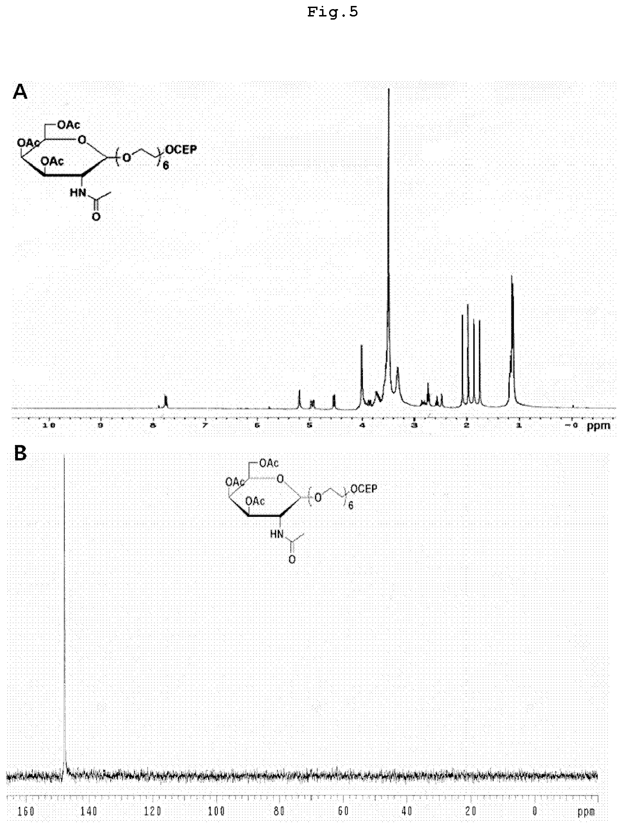

FIG. 5 shows the results of NMR analysis of 1-hexa (ethylene glycol) -NAG-phosphoramidite) (compound C). (A) ¹H NMR (300 MHz, DMSO-D₆); δ 7.78 ppm (1H, d, J=9.3 Hz), 5.21 ppm (1H, d, J=3.0 Hz), 4.97 ppm (1H, dd, J=11.1, 3.6 Hz), 4.56 ppm (1H, d, J=8.1 Hz), 3.88 ppm (1H, d, J=9.0 Hz), 3.81-3.41 ppm (30H, m), 2.89 ppm (2H, t, J=5.7 Hz), 2.11 ppm (3H, s), 2.00 ppm (3H, s), 1.89 ppm (3H, s), 1.77 ppm (3H, s), 1.20-1.12 ppm (12H, m), (B) ³¹P NMR data (121 MHz, DMSO-D₆); δ 147.32 ppm.

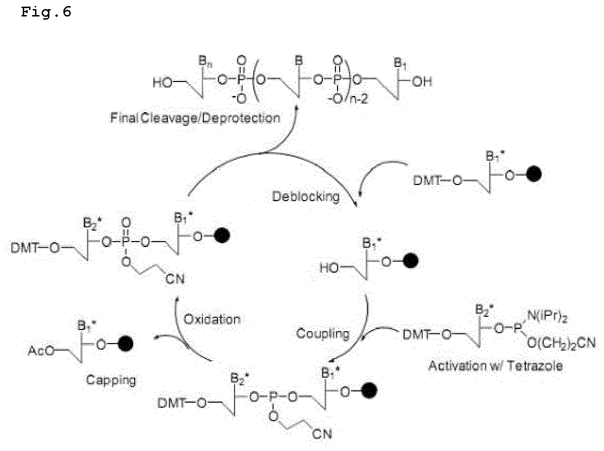

FIG. 6 shows a process of preparing a single-stranded RNA.

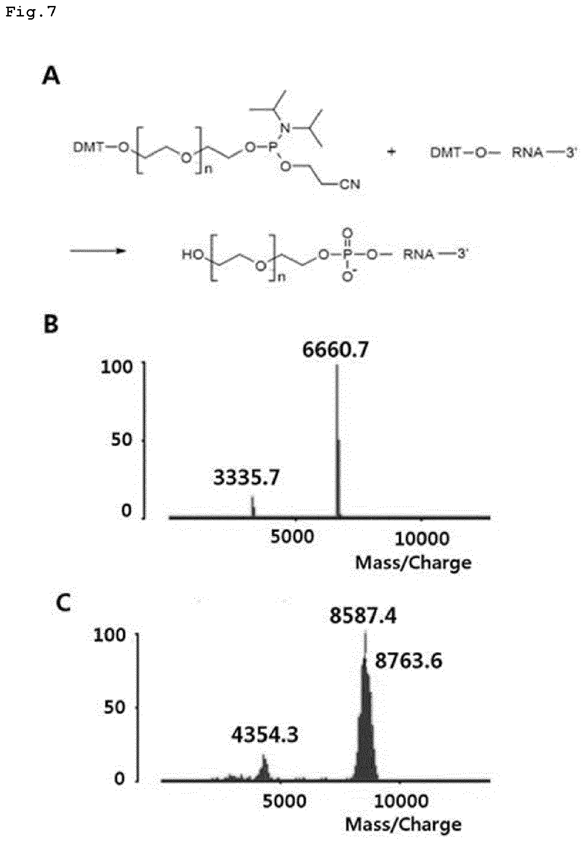

FIG. 7 shows a process for preparing a double-helix oligo RNA structure comprising PEG bonded to the 5' end of a double-helix oligo RNA and the results of analysis of the RNA structure. FIG. 7(A) shows a process of bonding PEG to a double-helix oligo RNA using PEG-phosphoramidite, FIG. 7(B) shows the results of MALDI-TOF MS analysis of a single-stranded RNA (21mer) which was not modified at 5' end (SEQ ID NO: 1; MW 6662.1), and FIG. 7(C) shows the results of MALDI-TOF MS of a single-stranded RNA (21mer) having PEG bound to the 5' end (SEQ ID NO: 1; MW 6662.1).

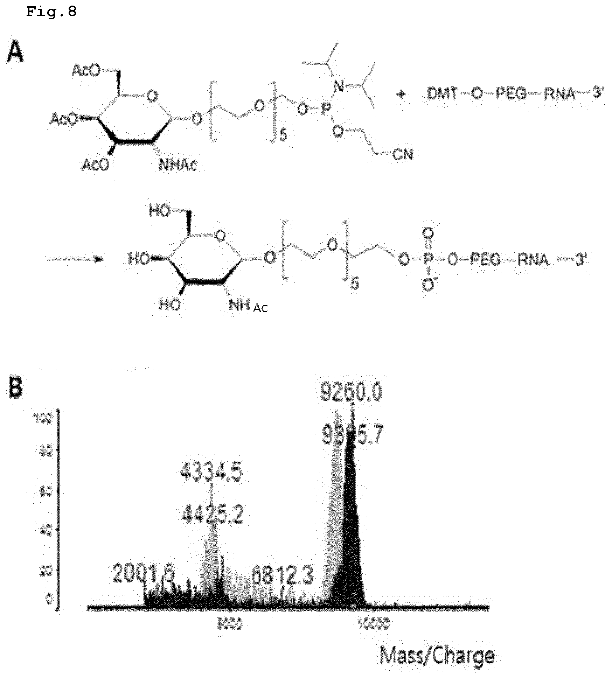

FIG. 8 shows a process for preparing a mono-NAG-PEG-RNA structure and the results of analysis of the structure. FIG. 8(A) shows a process of bonding *N*-acetyl galactosamine (NAG) to PEG-RNA using *N*-acetyl galactosamine-phosphoramidite, FIG. 8(B) shows the results of MALDI-TOF MS analysis of a NAG-PEG-RNA structure (blue, MW 9171.2) structure comprising *N*-acetyl galactosamine bonded to PEG-RNA (green, MW 8624.1) and shows that the central peak shifted by the molecular weight of N-acetyl galactosamine (MW 547).

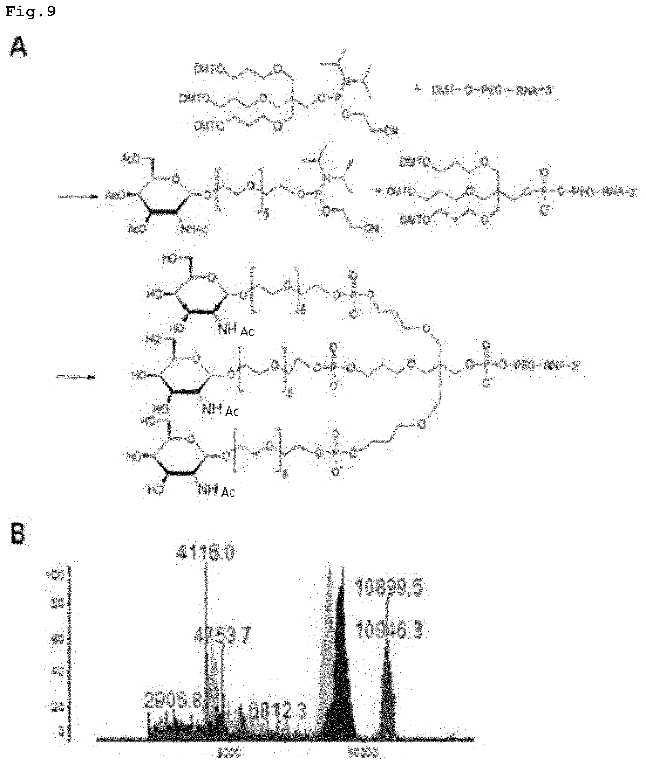

FIG. 9 shows a process for preparing a triple-NAG-PEG-RNA structure and the results of analysis of the structure. FIG. 9(A) shows a process of bonding *N*-acetyl galactosamine to PEG-RNA using dendrimer phosphoramidite and NAG-phosphoramidite, and FIG. 9(B) shows the results of MALDI-TOF MS analysis of PEG-RNA (green, M.W. 8624.1), and a mono-NAG-PEG structure (blue, MW 9171.2) and a triple-NAG-PEG-RNA structure (red, MW 10630), which comprise *N*-acetyl galactosamine bonded thereto.

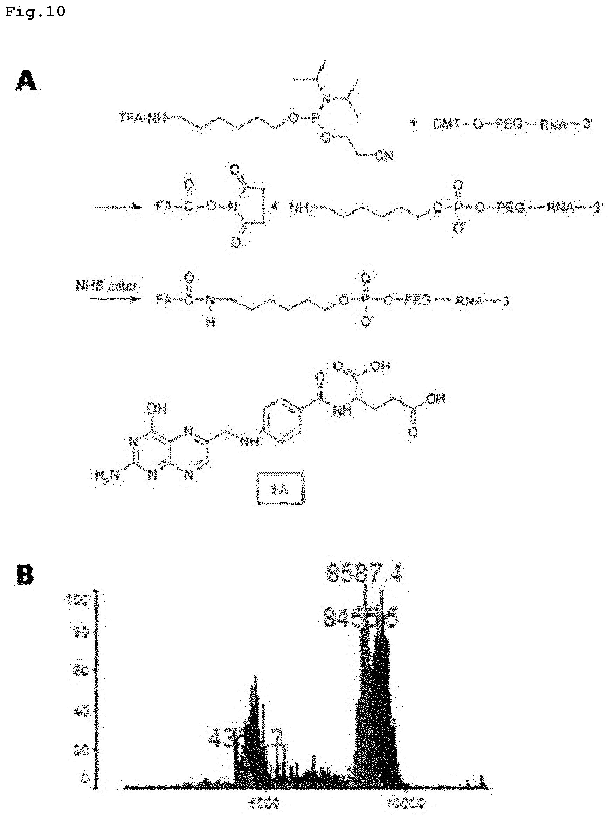

FIG. 10 shows a process for preparing a 5' folate-PEG-RNA structure and the results of analysis of the structure. FIG. 10(A) shows a process of bonding folate to PEG-RNA by NHS-folate, and FIG. 10(B) shows the results of MALDI-TOF MS analysis of PEG-RNA (green, MW 8624.1) and a folate-PEG-RNA structure (blue, MW 9277.8) and shows that the central peak shifted by the molecular weight of folate (MW 615).

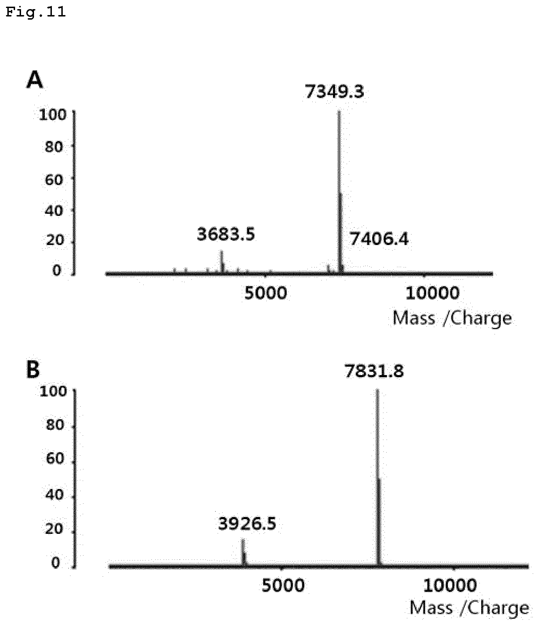

FIG. 11 shows the results of analysis of a 5' C₂₄-RNA structure. FIG. 11(A) shows the analysis of MALDI-TOF MS analysis of a single-stranded RNA complementary to SEQ ID NO: 1 (MW 7349.5), and FIG. 11(B) shows the analysis of MALDI-TOF MS analysis of a 5' C₂₄-RNA structure complementary to SEQ ID NO: 1 (MW 7830.2).

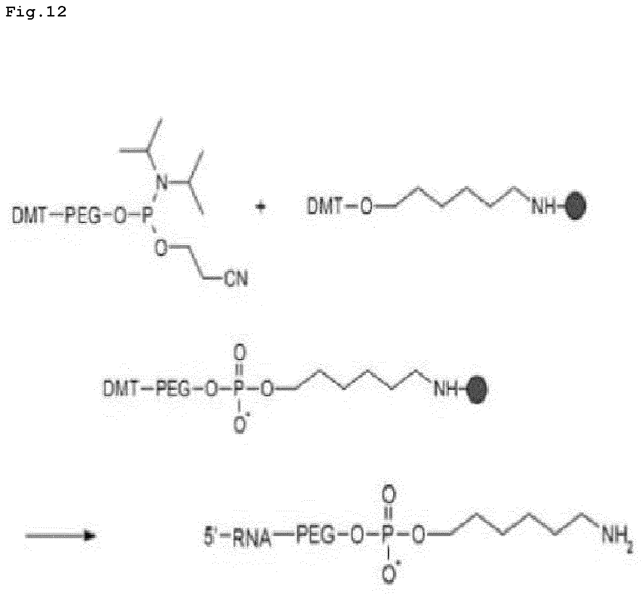

FIG. 12 shows a process of preparing a 3' CPG-amine-PEG-RNA structure by amine-CPG.

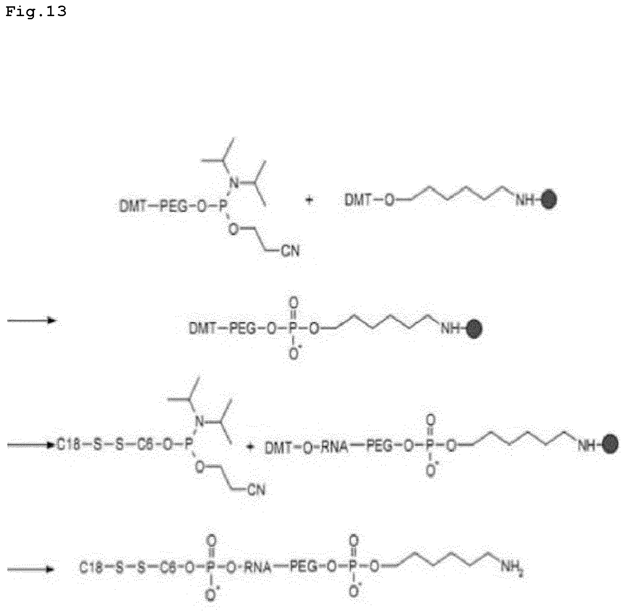

FIG. 13 shows a process of preparing a 3' CPG-amine-PEG-RNA-C₂₄ structure by amine-CPG.

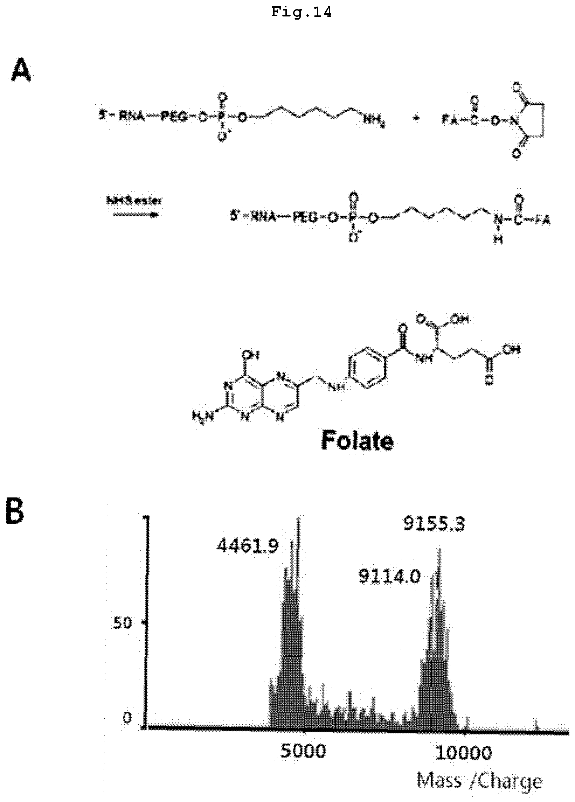

FIG. 14 shows a process of preparing a 3' folate-PEG-RNA structure and the results of analysis of the structure. FIG. 14(A) shows a process of bonding folate to a 3' amine-PEG-RNA structure by NHS-folate; and FIG. 14(B) shows the results of MALDI-TOF MS analysis of a 3' folate-PEG-RNA structure (SEQ ID NO: 1; MW 9277.7).

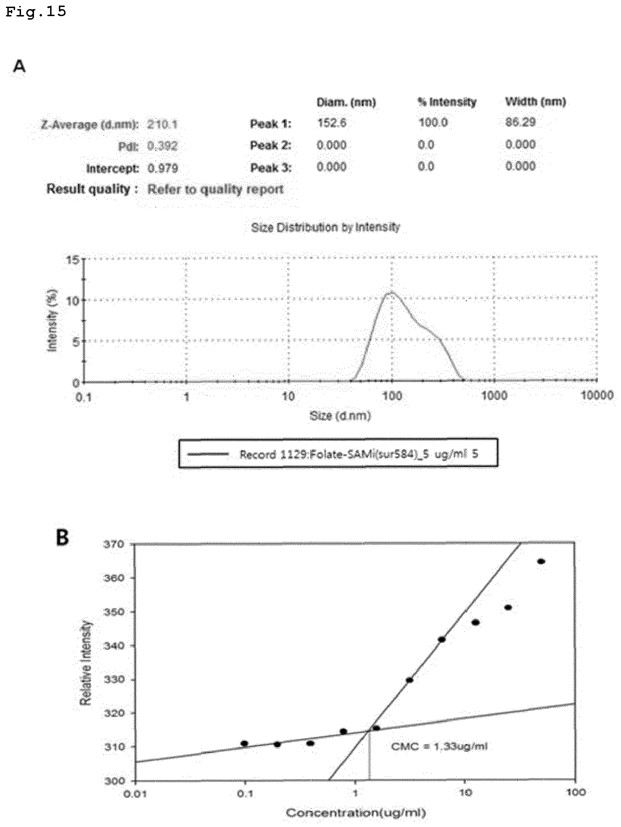

FIG. 15 shows the results of analyzing the physical properties of a nanoparticle (folate-SAMiRNA) composed of 5' folate-RNA-polymer structures. FIG. 15(A) is a graphic diagram showing the size and polydisperse index (PDI) of folate-SAMiRNA, and FIG. 15(B) is a graphic diagram showing the critical micelle concentration of folate-SAMiRNA.

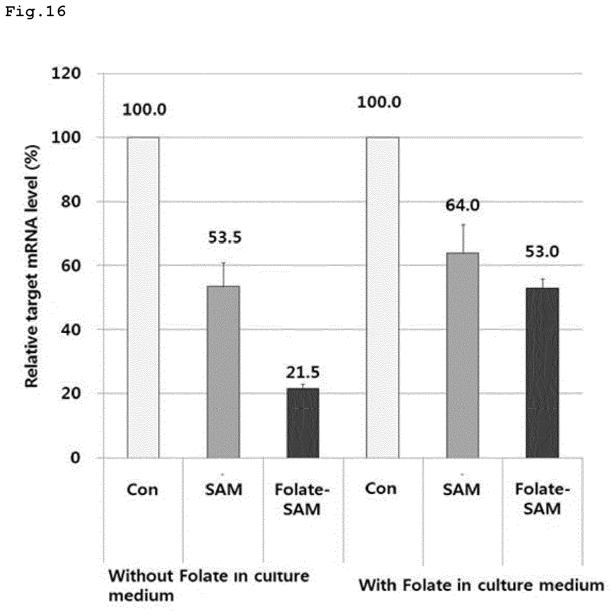

FIG. 16 shows the effect of folate-SAMiRNA on the inhibition of expression of the target gene in a cell line that overexpresses the folate receptor. The level of the target gene mRNA in the tumor cell line KB that overexpresses folate receptor was measured by qPCR at 48 hours after treatment with folate-SAMiRNA and SAMiRNA. In FIG. 16, Without Folate in culture medium: folate-free condition; With Folate in culture medium: a condition containing an excessive amount (1 mM) of folate; Con: a test group treated with the nanoparticle SAMiRNA-Con composed of double-helix oligo RNA-polymer structures comprising a sequence of SEQ ID NO: 2 (control sequence); SAM: a test group treated with the nanoparticle SAMiRNA-Sur composed of double-helix oligo RNA-polymer structures comprising a sequence of SEQ ID NO: 1 (survivin sequence); Folate-SAM: a test group treated with the nanoparticle Folate-SAMiRNA-Sur composed of folate-double stranded oligo RNAs comprising a sequence of SEQ ID NO: 1 (survivin sequence) and having a folate ligand bonded thereto.

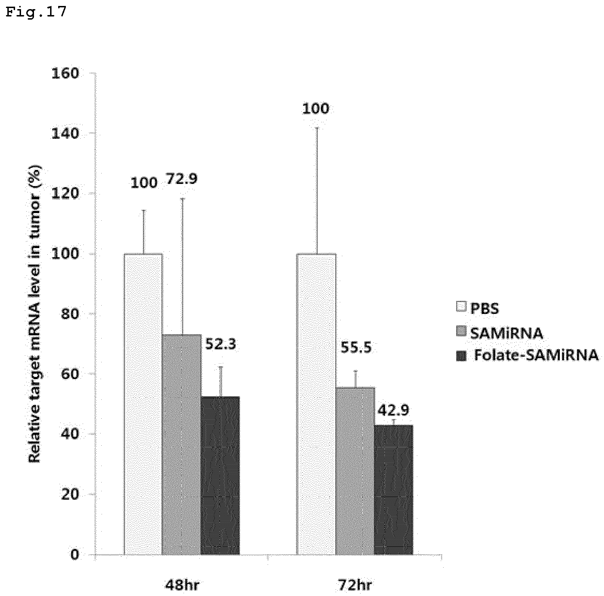

FIG. 17 shows the effect of folate-SAMiRNA on the inhibition of expression of the target gene in tumor tissue. The mRNA level of the target gene (survivin) in tumor tissue was measured by qPCR at 48 hours or 72 hours after each of SAMiRNA and folate-SAMiRNA was administered once at a dose of 5 mg/Kg body weight to the tail vein of a mouse having a tumor composed of the KB tumor cell line that overexpresses folate receptor 48. In FIG. 17, PBS: negative control; SAMiRNA: a group administered with the nanoparticle SAMiRNA-Sur composed of double-helix oligo RNA-polymer structures comprising a sequence of SEQ ID NO: 1 (survivin sequence) and having no ligand bonded thereto; Folate-SAMiRNA: a group administered with the nanoparticle Folate-SAMiRNA-Sur of SEQ ID NO: 1 (survivin sequence) having a folate ligand bonded thereto.

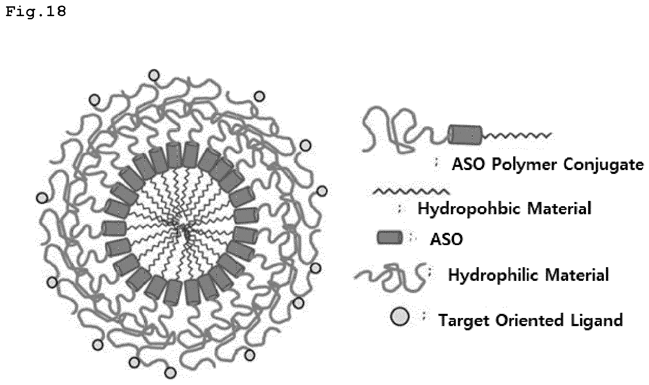

FIG. 18 is a schematic view of a nanoparticle comprising an ASO-polymer conjugate.

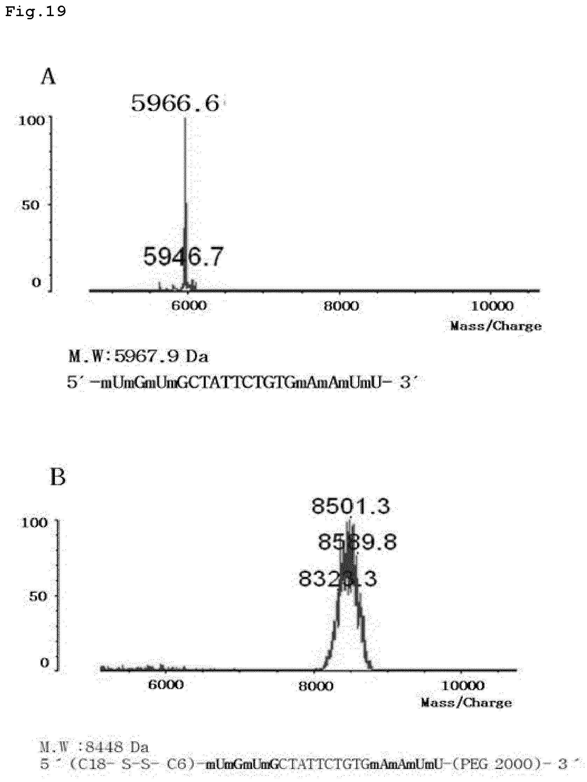

FIG. 19 shows the MALDI-TOF MS spectrum of each of an ASO and an ASO-polymer conjugate according to the present invention. Four nucleotides at both ends (5' and 3' end) are modified with 2-OCH₃ (methoxy), and 'm' indicates an OCH₃ (methoxy) group. FIG. 19(A) shows the MALDI-TOF data of the ASO (M.W. 5967.9 Da), and FIG. 19(B) shows the MALDI-TOF data of the ASO-polymer conjugate (M.W. 8448 Da).

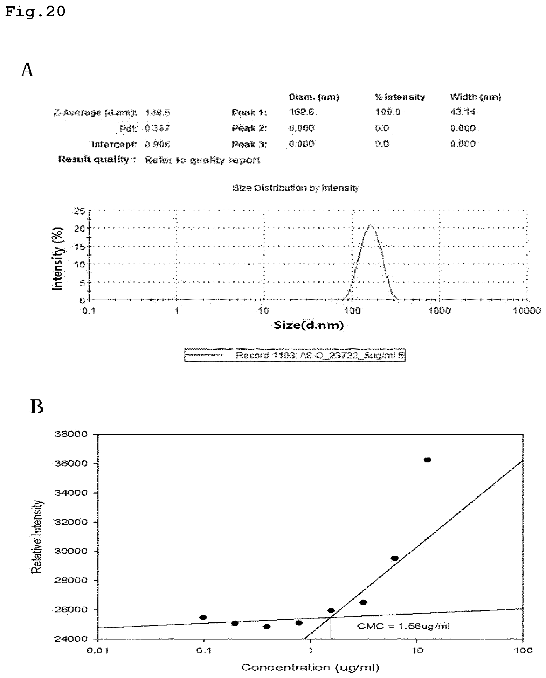

FIG. 20 shows the results of analyzing the physical properties of a nanoparticle composed of ASO-polymer conjugates. FIG. 20(A) is a graphic diagram showing the size and polydispersity index (PDI) of a nanoparticle composed of ASO-polymer conjugates; and FIG. 20(B) is a graphic diagram showing the critical micelle concentration of the nanoparticle composed of the ASO-polymer conjugates.

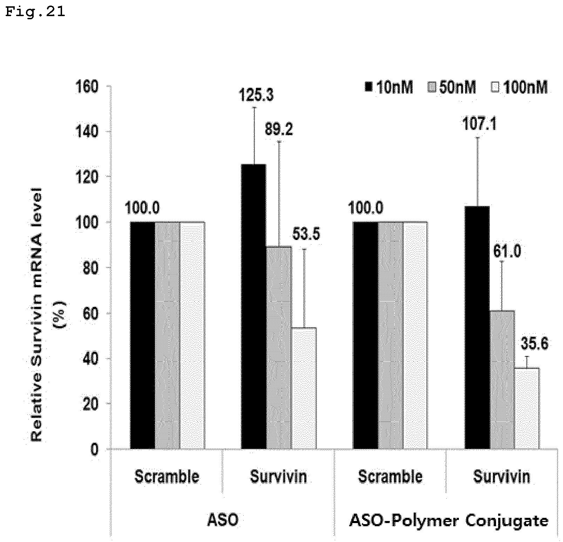

FIG. 21 shows the results of analyzing the mRNA expression level at various treatment concentrations (10, 50 and 100 nM) in order to examine the effects of an ASO and an ASO-polymer conjugate on the inhibition of expression of the target gene in tumor cells. Scramble: an ASO of SEQ ID NO: 4 (control sequence); Survivin: an ASO of SEQ ID NO: 3 (survivin sequence); ASO: an ASO having no material bonded thereto; ASO-polymer conjugate: an ASO-polymer conjugate in the form of 3'PEG-ASO-5'lipid).

### BEST MODE FOR CARRYING OUT THE INVENTION

### 1. First aspect of the present invention

In the first aspect of the present invention, the term "antisense strand" means a strand that shows RNAi activity to bind and degrade the target mRNA in RISC (RNA-induced silencing complex), and the term "sense strand" means a strand having a sequence complementary to the antisense strand.

As used herein, the term "complementary" or "complementary binding" means that two sequences to bind to each other to form a double-stranded structure. It includes not only a perfect match between two sequences, but also a mismatch between two sequences.

The present invention provides a therapeutic drug-polymer structure having a structure of the following formula (1) and comprising a ligand bonded thereto:

Formula 1 L-A-X-R-Y-B

wherein A is a hydrophilic material; B is a hydrophobic material; X and Y are each independently a simple covalent bond or a linker-mediated covalent bond; R is a therapeutic drug; and L is a receptor-specific ligand having the property of enhancing internalization of the target cell by receptor-mediated endocytosis (RME).

Herein, the therapeutic drug may be selected from among anticancer drugs, double-helix oligo RNAs, antiviral drugs, steroidal anti-inflammatory drugs (SAIDs), non-steroidal anti-inflammatory drugs (NSAIDs), antibiotics, antifungal agents, vitamins, hormones, retinoic acid, prostaglandins, prostacyclins, anti-metabolic agents, micotics, choline agonists, adrenalin antagonists, anticonvulsants, anti-anxiety drugs, tranquilizers, anti-depressants, anesthetics, analgesics, anabolic steroids, estrogens, progesterones, glycosaminoglycans, polynucleotides, immunosuppressants, and immunostimulants.

In the present invention, the therapeutic drug is preferably a double-helix oligo RNA or an anticancer drug. If the therapeutic drug is a double-helix oligo RNA, the hydrophilic material may be bonded to the 3' or 5' end of the double-helix oligo RNA.

In the inventive therapeutic drug-polymer structure comprising a ligand bonded thereto, a ligand may additionally be bonded to a specific position (particularly end) of the hydrophilic material bonded to the double-helix oligo RNA or the anticancer drug. The ligand may be selected from among a target receptor-specific antibody, an aptamer (a single-stranded nucleic acid (DNA, RNA or modified nucleic acid) capable of binding to the target molecule with high affinity and specificity), a peptide, or chemical materials, including folate (folate is used interchangeably with folic acid, and the term "folate" as used herein refers to folate that is active in nature or in the human body), *N*-Acetylgalactosamine (NAG) and mannose, which have the property of binding specifically to the receptor that enhances internalization of the target cell by RME. Herein, the ligand is a material that performs delivery in a target receptor-specific manner, and is not limited only to the above-described antibody, aptamer, peptide and chemical materials.

The double-helix oligo RNA is preferably composed of 19-31 nucleotides. The double-helix oligo RNA that is used in the present invention may be any double-helix oligo RNA for any gene which is used or can be used for gene therapy or research.

The hydrophobic material functions to form a nanoparticle composed of double-helix oligo RNA structures by hydrophobic interaction. Among the hydrophobic materials, a carbon chain or cholesterol is very suitable for use in the preparation of the structure of the present invention, because it can be easily bonded in the step of synthesizing double-helix oligo RNAs.

The hydrophobic material preferably has a molecular weight of 250-1,000. Particularly, the hydrophobic material that is used in the present invention may be a steroid derivative, a glyceride derivative, glycerol ether, polypropylene glycol, a C₁₂-C₅₀ unsaturated or saturated hydrocarbon, diacyl phosphatidylcholine, fatty acid, phospholipid, lipopolyamine or the like, but is not limited thereto, and it will be obvious to those skilled in the art that any hydrophobic material suitable for the purpose of the present invention may be used.

Particularly, the steroid derivative may be selected from the group consisting of cholesterol, cholestanol, cholic acid, cholesteryl formate, cholestanyl formate, and cholestanyl amine, and the glyceride derivative may be selected from among mono-, di- and tri-glycerides, wherein the fatty acid of the glyceride may be a C₁₂-C₅₀ unsaturated or saturated fatty acid.

Also, the hydrophilic material is preferably a cationic or non-ionic polymer having a molecular weight of 200-10,000, and more preferably a non-ionic polymer having a molecular weight of 1,000-2,000 For example, the hydrophilic material that is used in the present invention is preferably a non-ionic hydrophilic polymer compound such as polyethylene glycol, polyvinyl pyrolidone or polyoxazoline, but is not limited thereto.

The hydrophilic material may, if necessary, be modified to have a functional group required for bonding to the ligand. Among the hydrophilic materials, particularly polyethylene glycol (PEG) may have various molecular weights and functional groups, has good biocompatibility, induces no immune response, increases the *in vivo* stability of the double-helix oligo RNA, and increases the delivery efficiency of the RNA, and thus it is very suitable for the preparation of the inventive double-helix oligo RNA structure.

The linker that mediates the covalent bond is not specifically limited, as long as it forms a covalent bond between the hydrophilic material (or the hydrophobic material) and the end of the double-helix oligo RNA and provides a bond that can, if necessary, be degraded in a specific environment. Thus, the linker may include any compound that bonds the hydrophilic material (or the hydrophobic material) to the double-helix oligo RNA during the process of preparing the structure.

Also, the covalent bond may be a non-degradable bond or a degradable bond. Herein, the non-degradable bonds include, but are not limited to, an amide bond or a phosphate bone, and the degradable bonds include, but are not limited to, a disulfide bond, an acid-degradable bond, an ester bond, an anhydride bond, a biodegradable bond or an enzymatically degradable bond.

The present invention provides a ligand-conjugated double-helix oligo RNA structure represented by the following formula (2), which comprises a hydrophilic material bonded to the 3' end of the sense strand of a double-helix oligo RNA, a ligand bonded to the hydrophilic material, and a hydrophobic material bonded to the 5' end of the sense strand:

Formula 2 B - X - 5' S 3' - Y - A - L AS

wherein A is the hydrophilic material; B is the hydrophobic material; X and Y are each independently a simple covalent bond or a linker-mediated covalent bond; S is the sense strand of the double-helix oligo RNA; AS is the antisense strand of the double-helix oligo RNA; and L is the receptor-specific ligand having the property of enhancing internalization of the target cell by receptor-mediated endocytosis (RME).

A method for preparing the ligand-conjugated double-helix oligo RNA structure represented by formula (2) comprises the steps of:
(1) synthesizing a single-stranded RNA on a solid support having a functional group-hydrophilic material bonded thereto;
(2) covalently bonding a hydrophobic material to the 5' end of the single-stranded RNA having the functional group-hydrophilic material bonded thereto;
(4) separating the functional group-RNA-polymer structure and a separately synthesized complementary single-stranded RNA from the solid support;
(5) bonding a ligand to the end of the hydrophilic material by the functional group; and
(6) annealing the ligand-bonded RNA-polymer structure with the complementary single-stranded RNA to form a double-stranded RNA structure.

In a more preferred embodiment of the present invention, the method may comprise the steps of: (1) bonding a hydrophilic material to a solid support (CPG) having a functional group bonded thereto; (2) synthesizing a single-stranded RNA on the solid support (CPG) having the functional group-hydrophilic material bonded thereto; (3) covalently bonding a hydrophobic material to the 5' end of the single-stranded RNA; (4) separating the functional group-RNA-polymer structure and a separately synthesized complementary single-stranded RNA from the solid support (CPG); (5) bonding a ligand to the end of the hydrophilic material by the functional group to prepare an RNA-polymer structure having the ligand boned thereto; and (6) annealing the ligand-bonded RNA-polymer structure with the complementary single-stranded RNA to form a double-helix oligo RNA structure having the ligand bonded thereto. After step (6), the RNA-polymer structure and the complementary single-stranded RNA can be separated and purified from the reactants by high-performance liquid chromatography (HPLC), and then the molecular weight can be measured by a MALDI-TOF mass spectrometer to determine whether the desired RNA-polymer structure and RNA were prepared. In the above-described preparation method, the step of synthesizing the single-stranded RNA complementary to the single-stranded RNA synthesized in step (3) may be performed before step (1) or in any one of steps (1) to (6).

In another embodiment, the present invention provides a ligand-bonded double-helix oligo RNA structure represented by the following formula (3), which comprises a hydrophilic material bonded to the 5' end of the sense strand of a double-helix oligo RNA, a ligand bonded to the hydrophilic material, and a hydrophobic material bonded to the 3' end of the sense strand:

Formula 3 L - A - X - 5' S 3' - Y - B 3' AS 5'

A method for preparing the ligand-bonded double-helix oligo RNA structure represented by formula (3) comprises the steps of:
(1) synthesizing a single-stranded RNA on a solid support having a functional group bonded thereto;
(2) covalently bonding a hydrophilic material to the material obtained in step (1);
(3) covalently bonding a ligand to the material obtained in step (2);
(4) separating the material obtained in step (3) from the solid support;
(5) covalently bonding a hydrophobic material to the material resulting from step (4) by the functional group bonded to the 3' end; and
(6) annealing the material resulting from step (5) with a complementary single-stranded RNA to form a double-strand RNA structure.

In a more preferred embodiment, the preparation method may comprise the steps of: (1) synthesizing a single-stranded RNA on a solid support (CPG) having a functional group bonded thereto; (2) covalently bonding a hydrophilic material to the 5' end of the single stranded RNA; (3) bonding a ligand to the hydrophilic material bonded to the single-stranded RNA to synthesize a functional group-RNA-hydrophilic polymer structure; (4) separating functional group-RNA-hydrophilic polymer structure from the solid support (CPG); (5) bonding a hydrophobic material to the RNA via the functional group to synthesize an RNA-polymer structure having a ligand bonded thereto; and (6) annealing the prepared RNA-polymer structure with a complementary single-stranded RNA to prepare a double-helix oligo RNA-polymer structure.

After step (5), the RNA can be separated and purified from the reactants by high-performance liquid chromatography (HPLC), and then the molecular weight can be measured by a MALDI-TOF mass spectrometer to determine whether the desired RNA-polymer structure and RNA were prepared. In the above-described preparation method, the step of synthesizing the single-stranded RNA complementary to the single-stranded RNA synthesized in step (1) may be performed before step (1) or in any one of steps (1) to (6).

In another embodiment, the present invention provides a ligand-bonded double-helix oligo RNA structure represented by the following formula (4), which comprises a hydrophilic or hydrophobic material bonded to the 5' end of the sense strand and antisense strand of a double-helix RNA:

Formula (4) L - A - X - 5' S 3' 3' AS 5' - Y - B

wherein A is a hydrophilic material; B is a hydrophobic material; X and Y are each independently a simple covalent bond or a linker-mediated covalent bond; S is the sense strand of a double-helix oligo RNA; AS is the antisense strand of the double-helix oligo RNA; and L is a receptor-specific ligand having the property of enhancing internalization of the target cell by receptor-mediated endocytosis (RME).

A method for preparing the double-helix oligo RNA structure represented by formula (4) comprises the steps of:
(1) synthesizing a single-stranded RNA on a solid support;
(2) covalently bonding a hydrophilic material to the 5' end of the single-stranded RNA;
(3) bonding a ligand to the hydrophilic material bonded to the single-stranded RNA;
(4) separating the ligand-bonded, RNA-hydrophilic polymer structure and a separately synthesized complementary RNA-hydrophobic polymer structure from the solid support; and
(5) annealing the ligand-bonded, RNA-hydrophilic polymer structure with the complementary RNA-hydrophobic polymer structure to form a double-stranded structure.

The preparation method comprises, between steps (1) to (4), a step of synthesizing a single-stranded RNA complementary to the single-stranded RNA of step (1), and then covalently bonding a hydrophobic material to the synthesized single-stranded RNA to synthesize a single-stranded RNA-hydrophobic polymer structure.

The present invention also provides a nanoparticle comprising the double-helix oligo RNA structure having the ligand bonded thereto, and a nanoparticle comprising the therapeutic drug-polymer structure having the ligand bonded thereto.

A nanoparticle is formed by interaction between the ligand-bonded, double-helix oligo RNA structures of the present invention. Specifically, a nanoparticle is formed, which has a structure in which a hydrophobic material is located in the center of the nanoparticle, a double-helix oligo RNA is protected by an external hydrophilic material, and a ligand is located on the surface of the nanoparticle (see FIG. 1). The nanoparticle delivers the double-helix oligo RNA into a cell by the ligand, and thus delivers the RNA into a cell with increased efficiency. This nanoparticle can be used for the treatment of diseases . Synthesis of the structure and the characteristics, intracellular delivery efficiency and effects of a nanoparticle comprising the structure will be described in further detail in the Examples below.

The present invention also provides a gene therapy method that uses a nanoparticle composed of the ligand-bonded double-helix oligo RNA structures or the ligand-bonded, therapeutic drug-polymer structures.

Specifically, the present invention provides a therapeutic method comprising the steps of: preparing a nanoparticle composed of the ligand-bonded double-helix oligo RNA structures; and introducing the nanoparticle into the body of an animal.

The present invention also provides a pharmaceutical composition comprising a pharmaceutically effective amount of a nanoparticle composed of the ligand-bonded double-helix oligo RNA structures.

For administration, the composition of the present invention may comprise, in addition to the above-described active ingredient, at least one pharmaceutically acceptable carrier. The pharmaceutically acceptable carrier that may be used in the present invention should be compatible with the active ingredient of the present invention and may be physiological saline, sterile water, Ringer's solution, buffered saline, dextrose solution, maltodextrin solution, glycerol, ethanol, or a mixture of two or more thereof. In addition, the composition of the present invention may, if necessary, comprise other conventional additives, including antioxidants, buffers, and bacteriostatic agents. Further, the composition of the present invention may be formulated as injectable forms such as aqueous solutions, suspensions or emulsions with the aid of diluents, dispersants, surfactants, binders and lubricants. Particularly, the composition of the present invention is preferably provided as a lyophilized formulation. For preparation of lyophilized formulations, any conventional method known in the art may be used, and a stabilizer for lyophilization may also be added.

In addition, the composition of the present invention may be formulated into suitable dosage forms depending on the kind of disease or component according to a method known in the art or the method disclosed in Remington's pharmaceutical Science (Mack Publishing Company, Easton PA).

The dosage of the pharmaceutical composition of the present invention can be determined by those skilled in the art depending on the conditions of the patient and the severity of the disease. In addition, the composition of the present invention may be formulated in the form of powders, tablets, capsules, liquid, injection solutions, ointments, and syrups, and may be provided in unit dosage forms or multiple dosage forms, for example, sealed ampoules or vials.

The pharmaceutical composition of the present invention can be administered orally or parenterally. The pharmaceutical composition according to the present invention can be administered by various routes, including, but not limited to, oral, intravenous, intramuscular, intra-arterial, intramedullary, intradural, intracardial, transdermal, subcutaneous, intraperitoneal, gastrointestinal, sublingual, and local routes.

For such clinical administration, the pharmaceutical composition of the present invention can be formulated in suitable forms using a technique known in the art. The dose of the composition of the present invention may vary depending on various factors, such as a patient's body weight, age, sex, health condition and diet, the time and method of administration, excretion rate, and severity of a disease, and may be easily determined by a person of ordinary skill in the art.

### 2. Second aspect of the present invention

In a second aspect, the present invention provides an ASO-polymer conjugate represented by the following formula 5:

Formula 5 A-X-R-Y-B

wherein one of A and B is a hydrophilic material, the other one is a hydrophobic material, X and Y are each independently a simple covalent bond or a linker-mediated covalent bond, and R is an ASO.

As used herein, the term "ASO" is meant to include not only conventional antisense oligonucleotides that are used to inhibit the expression of mRNA, but also antagomirs that inhibit the functions of microRNA.

The hydrophilic material in formula (5) may have a target-specific ligand attached thereto. The target-specific ligand has the property of enhancing target-specific internalization by receptor-mediated endocytosis (RME) and may be selected from among target-specific antibodies or aptamers, peptides such as receptor-specific ligands, and chemical materials such as folate, N-acetylgalactosamine (NAG), and mannose. Herein, the targeting moiety is a material that performs delivery in a target-specific manner, and is not limited only to the above-described antibody, aptamer, peptide and chemical materials.

In the conjugate of the present invention, the ASO preferably comprises 10-50 oligonucleotides, more preferably 13-25 oligonucleotides.

In order to enhance the *in vivo* stability, the ASO includes oligodeoxynucleotides (ODNs) obtained by various modifications that provide nuclease resistance. The modification may be one or a combination of two or more selected from among the substitution of an -OH group at the 2' carbon position of the sugar moiety of one or more nucleotides with -CH₃ (methyl), -OCH₃, -NH₂, -F(fluorine), -O-2-methoxyethyl, -O-propyl, -O-2-methylthioethyl, -O-3-aminopropyl, -O-3-dimethylaminopropyl, -O-*N*-methylacetamido or -O-dimethylamidoxyethyl; the substitution of oxygen of the sugar moiety of the nucleotide with sulfur; modification of the bond between the nucleotides into a phosphorothioate, boranophosphophate or methyl phosphonate bond. Alternatively, the modification may be modification in the form of PNA (peptide nucleic acid) or LNA (locked nucleic acid).

An ASO that may be used in the present invention is not specifically limited and may be an ASO for any gene which is used or can be used for gene therapy or research.

It will be obvious to those skilled in the art that the ASOs that are used in the present invention include not only an ASO having a perfect match with the target mRNA, but also an ASO that mismatches the target mRNA to inhibit the translation of the mRNA.

The hydrophilic material preferably has a molecular weight of 200-10,000, more preferably 1,000-2,000. Also, the hydrophilic material is preferably a cationic or nonionic polymer compound.

For example, the hydrophilic polymer compound that is used in the present invention is preferably a nonionic hydrophilic polymer compound such as PEG (polyethylene), polyvinylpyrolidone or polyoxazoline, but is not limited thereto.

The hydrophilic material may, if necessary, be modified to have a functional group required for bonding to other materials. Among the hydrophilic materials, particularly polyethylene glycol (PEG) may have various molecular weights and functional groups, has good biocompatibility, induces no immune response, increases the *in vivo* stability of the ASO, and increases the delivery efficiency of the ASO, and thus it is very suitable for the preparation of the inventive conjugate.

In addition, the hydrophobic material preferably has a molecular weight of 250-1,000. Particularly, the hydrophobic material that is used in the present invention may preferably be a steroid derivative, a glyceride derivative, glycerol ether, polypropylene glycol, a C₁₂-C₅₀ unsaturated or saturated hydrocarbon, diacyl phosphatidylcholine, fatty acid, phospholipid, lipopolyamine or the like, but is not limited thereto, and it will be obvious to those skilled in the art that any hydrophobic material suitable for the purpose of the present invention may be used.

Particularly, the steroid derivative may be selected from the group consisting of cholesterol, cholestanol, cholic acid, cholesteryl formate, cholestanyl formate, and cholestanyl amine, and the glyceride derivative may be selected from among mono-, di- and tri-glycerides, wherein the fatty acid of the glyceride may be a C₁₂-D₅₀ unsaturated or saturated fatty acid.

The hydrophobic material functions to cause a hydrophobic interaction to form a nanoparticle. Among the hydrophobic materials, particularly a carbon chain or cholesterol is very suitable for the preparation of the conjugate of the present invention, because it can be easily bonded in the step of preparing an ASO.

Also, the covalent bond indicated by X or Y in formula 5 may be a non-degradable bond or a degradable bond. Herein, the non-degradable bonds include, but are not limited to, an amide bond or a phosphate bone, and the degradable bonds include, but are not limited to, a disulfide bond, an acid-degradable bond, an ester bond, an anhydride bond, a biodegradable bond or an enzymatically degradable bond.

An ASO-polymer conjugate according to the present invention may have a structure in which a hydrophilic material is bonded to one of the 5' and 3' ends of an ASO and a hydrophobic material is bonded to the other end.

A method for preparing the ASO-polymer conjugate having the hydrophilic material bonded to the 3' end of the ASO may comprises the steps of:
(a) covalently bonding a hydrophilic material to a solid support;
(b) synthesizing an ASO on the solid support comprising the hydrophilic material;
(c) covalently bonding a hydrophobic material to the 5' end of the ASO on the solid support; and
(d) separating and purifying the resulting ASO-polymer conjugate from the solid support.

In a more preferred embodiment, the ASO-polymer conjugate is prepared by a method comprising the steps of: covalently bonding a hydrophilic material to a solid support (Controlled Pore Glass (CPG)) ; synthesizing an ASO on the solid support (CPG), which has the hydrophilic material covalently bonded thereto, by deblocking, coupling, capping and oxidation; and covalently bonding a hydrophobic material to the 5' end of the ASO. After completion of the preparation of the ASO-polymer conjugate, the ASO-polymer conjugate is separated from the solid support (CPG) by treating it with 28%(v/v) ammonia in water bath at 60 °C, and the ASO-polymer conjugate can be separated and purified from the reactants by high-performance liquid chromatography (HPLC), after which the molecular weight may be measured by the MALDI-TOF mass spectrometer to determine whether the desired ASO-polymer conjugate was prepared.

In another aspect, a method for preparing an ASO-polymer conjugate comprising a hydrophilic material bonded to the 5' end of an ASO comprises the steps of:
(a) synthesizing an ASO on a solid support having a functional group bonded thereto;
(b) covalently bonding a hydrophilic material to the 5' end of the ASO;
(c) separating the hydrophilic material-bonded ASO conjugate from the solid support; and
(d) covalently bonding a hydrophobic material to the 3' end of the ASO separated from the solid support.

In a more preferred embodiment, the preparation method comprises the steps of: synthesizing an ASO on a solid support (CPG) having a functional group bonded thereto; covalently bonding a hydrophilic material to the 5' end of the ASO; treating the resulting solid support with 28 %(v/v) ammonia in water bath at 60 °C to separate the functional group-attached ASO-hydrophilic polymer conjugate from the solid support (CPG); and attaching a hydrophobic material to the ASO via the functional group to form an ASO-polymer conjugate comprising the hydrophilic material and hydrophobic material attached to both ends of the ASO. When the preparation of the ASO-polymer conjugate has been completed, the ASO-polymer conjugate can be separated and purified from the reactants by high-performance liquid chromatography (HPLC), after which the molecular weight may be measured by the MALDI-TOF mass spectrometer to determine whether the desired ASO-polymer conjugate was prepared.

Meanwhile, the ASO-polymer conjugate may further comprise a ligand bonded to the hydrophilic material.

A method of bonding a ligand to the hydrophilic material is determined according to the kind of functional group attached to the ligand. For example, a ligand-phosphoramidite having phosphoramidite as a functional group can be bonded to the hydrophilic material in the same manner as the ASO synthesis process, and a ligand having N-Hydroxysuccinimide (NHS) attached thereto can be bonded to the hydrophilic material by an N-Hydroxysuccinimide (NHS) ester bond.

A method for preparing an ASO-polymer conjugate comprising a ligand attached to an ASO-polymer conjugate having a hydrophilic material attached to the 3' end of an ASO comprises the steps of:
(a) bonding a hydrophilic material to a solid support having a functional group attached thereto;
(b) synthesizing an ASO on the solid support having the functional group-hydrophilic material bonded thereto;
(c) covalently bonding a hydrophobic material to the 5' end of the ASO;
(d) separating an ASO-polymer conjugate, obtained in step (c), from the solid support; and
(e) bonding a ligand to the hydrophilic material of the ASO-polymer conjugate separated from the solid support.

In a more preferred embodiment, the preparation method comprises the steps of: bonding a hydrophilic polymer to a solid support having a functional group attached thereto; synthesizing an ASO on the solid support (CPG) having the functional group-hydrophilic material bonded thereto; covalently bonding a hydrophobic material to the end group of the ASO; separating the resulting functional group-ASO-polymer conjugate from the solid support (CPG); and attaching a ligand to the end of hydrophilic polymer by the functional group, thereby preparing a ligand-bonded ASO-polymer conjugate. When the preparation of the ligand-bonded ASO-polymer conjugate has been completed, the ASO-polymer conjugate can be separated and purified from the reactants by high-performance liquid chromatography (HPLC), after which the molecular weight may be measured by the MALDI-TOF mass spectrometer to determine whether the desired ligand-bonded ASO-polymer conjugate was prepared.

In another aspect, a method for preparing an ASO-polymer conjugate comprising a ligand attached to an ASO-polymer conjugate having a hydrophilic material attached to the 5' end of an ASO comprises the steps of:
(a) synthesizing an ASO on a solid support having a functional group attached thereto;
(b) covalently bonding a hydrophilic material to the end of the ASO;
(c) covalently bonding a ligand to the ASO-hydrophilic material conjugate;
(d) separating an ASO-hydrophilic material-ligand conjugate, which has the functional group attached thereto, from the solid support; and
(e) covalently bonding a hydrophobic material to the 3' end of the ASO of the conjugate separated from the solid conjugate.

In a more preferred embodiment, the preparation method comprises the steps of: synthesizing an ASO on a solid support (CPG) having a functional group attached thereto; covalently binding a hydrophilic material to the end group of the ASO; covalently bonding a ligand to the ASO-hydrophilic polymer; separating the functional group-attached ASO-hydrophilic polymer-ligand conjugate from the solid support (CPG); and attaching a hydrophobic material to the separated conjugate by the functional group, thereby synthesizing a ligand-bonded ASO-polymer conjugate having the hydrophobic material attached to the end opposite the hydrophilic polymer. When the preparation of the ligand-bonded ASO-polymer conjugate has been completed, the ASO-polymer conjugate can be separated and purified from the reactants by high-performance liquid chromatography (HPLC), after which the molecular weight may be measured by the MALDI-TOF mass spectrometer to determine whether the desired ligand-bonded ASO-polymer conjugate was prepared.

As a result, the ASO-polymer conjugate synthesized in the present invention comprises both hydrophobic and hydrophilic materials, and thus is amphiphilic in nature. The hydrophilic moiety tends to go outward by interaction (such as hydrogen bond) with water molecules *in vivo,* and the hydrophobic material tends to go inward by hydrophobic interaction, and thus a thermodynamically stable nanoparticle is formed. In other words, a nanoparticle is formed in which the hydrophobic material is located in the center of the nanoparticle and the hydrophilic material is located outside the ASO to protect the ASO (see FIG. 18). The nanoparticle formed as described above enhances the intracellular delivery of the ASO and can be used for the treatment of diseases. Synthesis of the conjugate and the characteristics, intracellular delivery efficiency and effects of the conjugate will be described in further detail in the Examples below.

In addition, the present invention provides a gene therapy method comprising the steps of: preparing a nanoparticle composed of the ASO-polymer conjugates; and delivering the ASO *in vitro* by the nanoparticle. The gene therapy method is not limited only to application *in vitro.*

The present invention also provides a pharmaceutical composition comprising a pharmaceutically effective amount of the ASO-polymer conjugate or a nanoparticle composed of the ligand-bonded ASO-polymer conjugate.

For administration, the composition of the present invention may comprise, in addition to the above-described active ingredient, at least one pharmaceutically acceptable carrier. The pharmaceutically acceptable carrier that may be used in the present invention should be compatible with the active ingredient of the present invention and may be physiological saline, sterile water, Ringer's solution, buffered saline, dextrose solution, maltodextrin solution, glycerol, ethanol, or a mixture of two or more thereof. In addition, the composition of the present invention may, if necessary, comprise other conventional additives, including antioxidants, buffers, and bacteriostatic agents. Further, the composition of the present invention may be formulated as injectable forms such as aqueous solutions, suspensions or emulsions with the aid of diluents, dispersants, surfactants, binders and lubricants. Particularly, the composition of the present invention is preferably provided as a lyophilized formulation. For preparation of lyophilized formulations, any conventional method known in the art may be used, and a stabilizer for lyophilization may also be added.

In addition, the composition of the present invention may be formulated into suitable dosage forms depending on the kind of disease or component according to a method known in the art or the method disclosed in Remington's pharmaceutical Science (Mack Publishing Company, Easton PA).

The dosage of the pharmaceutical composition of the present invention can be determined by those skilled in the art depending on the conditions of the patient and the severity of the disease. In addition, the composition of the present invention may be formulated in the form of powders, tablets, capsules, liquid, injection solutions, ointments, and syrups, and may be provided in unit dosage forms or multiple dosage forms, for example, sealed ampoules or vials.

The pharmaceutical composition of the present invention can be administered orally or parenterally. The pharmaceutical composition according to the present invention can be administered by various routes, including, but not limited to, oral, intravenous, intramuscular, intra-arterial, intramedullary, intradural, intracardial, transdermal, subcutaneous, intraperitoneal, gastrointestinal, sublingual, and local routes. The dose of the composition of the present invention may vary depending on various factors, such as a patient's body weight, age, sex, health condition and diet, the time and method of administration, excretion rate, and severity of a disease, and may be easily determined by a person of ordinary skill in the art.

### EXAMPLES

Hereinafter, the present invention will be described in further detail with reference to examples. It will be obvious to a person having ordinary skill in the art that these examples are illustrative purposes only and are not to be construed to limit the scope of the present invention.

### Example 1: Preparation of ligand material that can be bonded

In order to prepare a double-helix oligo RNA structure having a ligand bonded thereto, a ligand material that can be bonded to the double-helix oligo RNA structure was prepared.

### Example 1-1: Preparation of 1-hexa(ethylene glycol)-N-acetyl galactosamine-phosphoramidite reagent (compound A, B and C)

In order to bond *N*-acetyl galactosamine (NAG) to a double-helix oligo RNA structure, 1-hexa(ethylene glycol)-NAG-phosphoramidite was prepared as shown in the following reaction scheme 1.

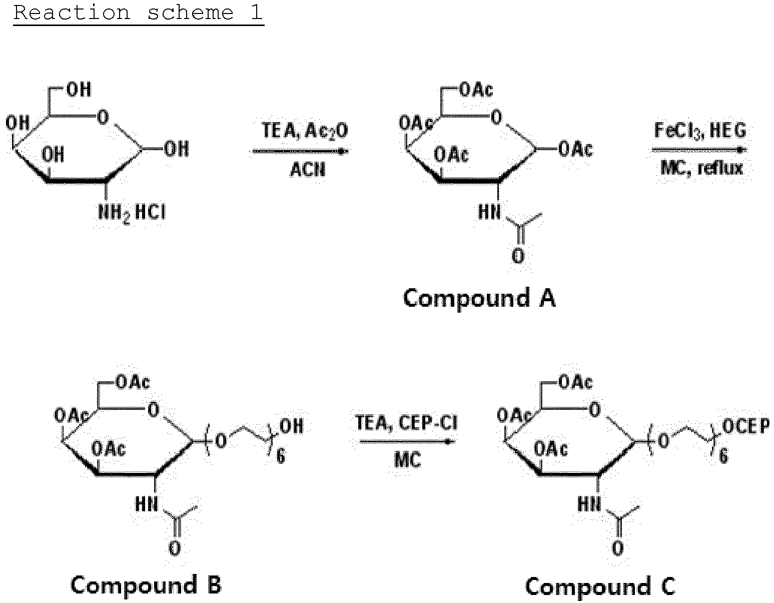

### Example 1-1-1: Preparation of 1,3,4,6-tetraacetyl-NAG (compound A)

The starting material galactosamine hydrochloride (Sigma Aldrich, USA) (2 g, 9.27 mmol), acetonitrile (Samjeon, Korea) (31 **Mℓ**) and triethylamine (Sigma Aldrich, USA) (15.42 **Mℓ,** 111.24 mmol) were mixed with each other and refluxed for 1 hour. The mixture was cooled slowly to room temperature and cooled to 0 **°C** using ice water, and then acetic anhydride (Sigma Aldrich, USA) (8.76 **Mℓ,** 92.70 mmol) was added dropwise thereto for 10 minutes. Then, the ice water was removed, and the remaining material was stirred at room temperature for 24 hours. After completion of the reaction, an aqueous solution of sodium bicarbonate (Samjeon, Korea) was added slowly to the reaction product until the pH reached neutral. After the pH reached neutral, the reaction solution was stirred at room temperature for 2 hours, and the produced solid was filtered. The filtrate was washed sequentially with ethyl acetate (Samjeon, Korea) (100 **Mℓ** × 2), distilled water (100 **Mℓ** × 2) and ethyl acetate (100 **Mℓ** × 1). The solid was vacuum-dried to yield 1,3,4,6-tetraacetyl-*N*-acetyl galactosamine (1.82 g, 52.3 %) (see FIG. 3).

### Example 1-1-2: Preparation of 3,4,6-triacetyl-1-hexa(ethylene glycol)-NAG (compound B)

The 1,3,4, 6-tetraacetyl-N-acetyl galactosamine (1.81 g, 4.82 mmol) prepared in Example 1-1-1, iron III chloride (Sigma Aldrich, USA) (1.02 g, 6.27 mmol) and methylene chloride (Samjeon, Korea) (48 **Mℓ**) were mixed with each other and stirred at room temperature for 10 minutes. Then, hexa(ethylene glycol) (Sigma Aldrich, USA) (1.58 **Mℓ,** 4.82 mmol) was added to the mixture, followed by reflux for 2 hours. After completion of the reaction, the reaction solution was filtered through celite (Sigma Aldrich, USA), and the filtrate was washed with methylene chloride (50 **Mℓ** × 2). The filtrate was concentrated under reduced pressure and added to ethyl acetate (100 **Mℓ**) and distilled water (100 **Mℓ**)**,** and the aqueous layer was collected. The collected aqueous layer was extracted with methylene chloride (100 **Mℓ** × 3), and the organic layer was collected, dried with anhydrous magnesium sulfate (Samjeon, Korea) and filtered. The filtrate was concentrated under reduced pressure and dried in a vacuum, thereby obtaining 3,4,6-triacetyl-1-hexa(ethylene glycol)-N-acetyl galactosamine (2.24 g, 749 %) (see FIG. 4).

### Example 1-1-3: Preparation of 1-hexa(ethylene glycol)-NAG-phosphoramidite (compound C)

The compound (2.22 g, 3.71 mmol) obtained in Example 1-1-2, methylene chloride (37 **Mℓ**) and triethylamine (0.94 **Mℓ,** 6.75 mmol) were mixed with each other and stirred at room temperature for 10 minutes. Then, 2-cyanoethyl *N*,*N-*diisopropylchlorophosphoramidite (Sigma Aldrich, USA) (0.75 **Mℓ,** 3.38 mmol) was added to the mixture and stirred for 45 minutes. After completion of the reaction, the reaction solution was concentrated under reduced pressure, and ethyl acetate (100 **Mℓ**) and distilled water (100 **Mℓ**) were added thereto. The organic layer was collected, dried with anhydrous magnesium sulfate and filtered. The filtrate was concentrated under reduced pressure and purified by column chromatography, thereby obtaining 1-hexa(ethylene glycol)-N-acetyl galactosamine-phosphoramidite (1.14 g, 42.2 %) (see FIG. 5).

### Example 1-2: Preparation of NHS-folate

In order to bond folate to a double-helix oligo RNA structure, NHS-folate was prepared as shown in the following reaction scheme 2:

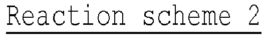

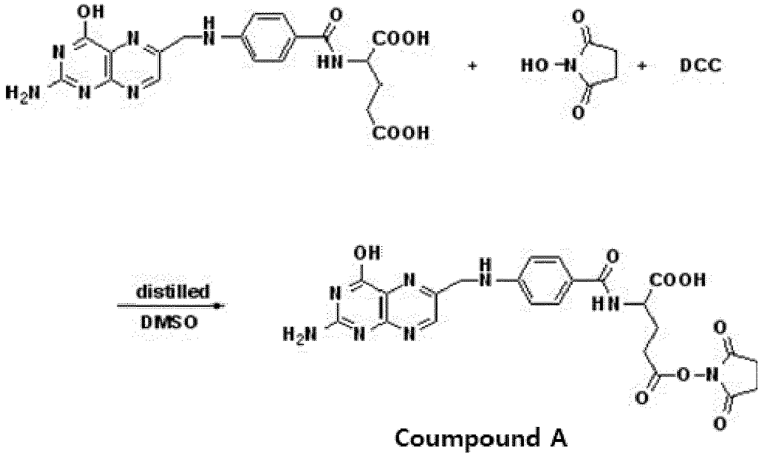

The starting material folic acid (Sigma Aldrich, USA) (3 g, 6.8 mmol), dimethyl sulfoxide (Sigma Aldrich, USA) (60 **Mℓ**), N-hydroxysuccinimide (Sigma Aldrich, USA) (0.86 g, 7.5 mmol) and 1,3-dicyclohexylcarbodiimide (Sigma Aldrich, USA) (154 g, 7.5 mmol) were mixed with each other and stirred at room temperature for 18 hours. After completion of the reaction, the reaction mixture was added dropwise to 950 ml of a 3:5 mixture of ethyl acetate: n-hexane (Samjeon, Korea) for 10 minutes, and the produced solid NHS-folate (3.79 g) was filtered (Robert J. Lee and Philip S. Low (1994) J. Biological Chemistry. 269: 3198-3204).

### Example 1-3: Preparation of peptide

Because peptide compounds include α-amino acid, a binding reaction between a peptide derivative having this structure and the amine functional group of PEG was performed. In this binding reaction, the amine functional groups present in PEG and the peptide derivative interact with each other during the binding reaction with the carboxylic acid of the peptide, and for this reason, a process of substituting the amine functional group of the peptide compound with a protecting group was required before the binding reaction. The amine group of the peptide compound was substituted with a protecting group of 9-fluorenylmethyloxycarbonyl (Fmoc) or tert-butyloxycarbonyl (t-BOC) to remove it reactivity with the carboxylic acid of the peptide, thereby preparing a peptide compound capable of binding to PEG.

### Example 2: Preparation of double-helix oligo RNA structure having ligand bonded to 5' end

The ligand material prepared in Example 1 can be constructed in the form of phosphoramidite such as the NAG-phosphoramidite of Example 1-1 so that it can be bonded to the end of PEG by a general chemical oligo synthesis process (consisting of deblocking, coupling, capping and oxidation). Alternatively, it can be constructed in the form of NHS-ligand such as the NHS-folate of Example 1-2 so that it can be bonded to amine-bonded PEG by an ester bond, thereby synthesizing a PEG-RNA structure having the ligand bonded to the 5' end of the RNA. The synthesized PEG-RNA structure having the ligand bonded to the 5' end was annealed with a complementary 5' C₂₄-RNA structure having a hydrophobic group bonded thereto, thereby synthesizing a double-helix oligo RNA structure having the ligand bonded to the 5' end.

### Example 2-1: Preparation of double-helix oligo RNA

In the following examples, a double-helix oligo RNA against survivin was used in order to inhibit survivin. Survivin is a protein that is expressed commonly in most tumors or mutant cell lines tested to date and is expected to be an important target in anticancer therapy (Survivin: a new target for anti-cancer therapy Cancer Treat Rev. 2009 Nov;35(7):553-62) The survivin double-helix oligo RNA according to the present invention consists of a sense strand set forth in SEQ ID NO: 1 and an antisense strand complementary thereto, and a double-stranded oligo nucleotide that is used as a control consists of a sense strand set forth in SEQ ID NO: 2 and an antisense strand complementary thereto. The double-helix oligo RNA used in this example consists of the following nucleotide sequence.
(SEQ ID NO: 1) 5'-AAG GAG AUC AAC AUU UUC A-3'
(SEQ ID NO: 2) 5'-CUU ACG CUG AGU ACU UCG A-3'

In order to synthesize the double-helix oligo RNA, the single-stranded RNA was synthesized by linking nucleotides by the phosphodiester bonds of the RNA backbone using tert-butyldimethylsilyl-protected β-cyanoethyl phosphoramidite (Polymer support oligonucleotide synthesis **XVIII:** use of β-cyanoethyl-*N,N*-dialkylamino-/*N*-morpholino phosphoramidite of deoxynucleosides for the synthesis of DNA fragments simplifying deprotection and isolation of the final product. Nucleic Acids Res. 1984 Jun 11; 12(11): 4539-57).

In the synthesis process, a cycle consisting of deblocking, coupling, capping and oxidation was repeated on a solid support having a nucleoside attached thereto, thereby obtaining a desired RNA sequence The process of synthesizing the single-stranded RNA was performed using a RNA synthesizer (384 Synthesizer, BIONEER, Korea)(see FIG. 6).

### Example 2-2: Preparation of 5' PEG-RNA structure

The RNA synthesized in Example 2-1 was reacted with a PEG phosphoramidite reagent according to a general RNA synthesis process, thereby preparing a 5' PEG-RNA structure (see FIG. 7).

### Example 2-3: Synthesis of PEG-RNA having ligand bonded to the 5' end

### Example 2-3-1: Preparation of NAG-PEG-RNA structure using N-acetyl galactosamine (NAG) phosphoramidite

The 5' PEG-RNA structure synthesized in Example 2-2 was reacted with the NAG phosphoramidite reagent (synthesized in Example 1-1) according to a general RNA synthesis process to bond *N*-acetyl galactosamine (NAG) to the PEG-RNA structure by a phosphodiester bond. The N-acetyl galactosamine ligand can provide one or more molecules of *N*-acetyl galactosamine to PEG using a dendrimer linker.

### Example 2-3-1-1: Preparation of mono NAG-PEG-RNA structure

The PEG-RNA structure synthesized in Example 2-2 was reacted with the NAG phosphoramidite reagent (synthesized in Example 1-1) according to a general RNA synthesis process to bond *N*-acetyl galactosamine (NAG) to the PEG-RNA structure by a phosphodiester bond, thereby synthesizing a 5' mono-NAG-PEG RNA structure (see FIG. 8).

### Example 2-3-1-2: Preparation of triple NAG-PEG-RNA structure

The PEG-RNA structure synthesized in Example 2-2 was reacted with a dendrimer phosphoramdite (Trebler Phosphoramidte, Glen research, USA) reagent, and then reacted with the NAG phosphoramidite reagent (synthesized in Example 1-1) according to a general RNA synthesis process to bond three NAGs to the PEG-RNA structure via phosphodiester bonds, thereby synthesizing a 5' triple-NAG-PEG-RNA structure (see FIG. 9).

### Example 2-3-2: Preparation of folate-PEG-RNA structure

The PEG-RNA structure synthesized in Example 2-2 was reacted with an amine phosphoramidite reagent according to a general RNA synthesis process to bond an amine group to the PEG-RNA structure via a phosphodiester bond, thereby synthesizing an amine-PEG-RNA structure. The synthesized amine-PEG-RNA structure was linked with the NHS-folate (synthesized in Example 1-2) via an ester bond to synthesize a 5' folate-PEG-RNA structure (see FIG. 10).

### Example 2-3-3: Preparation of peptide- PEG-RNA structure by amine modification and peptide compound

The binding reaction between the carboxyl group of the protected peptide compound prepared in Example 1-3 and the amine group of PEG of the amine-PEG-RNA structure prepared in Example 2-3-2 was performed using BOP (benzotriazole-1-yl-oxy-tris-(dimethylamino)-phosphonium hexafluorophosphate) (Sigma Aldrich, USA) and HOBT (1-hydroxybenzotriazole) (Sigma Aldrich, USA). After the binding reaction, the reaction product was treated with piperidine (Sigma Aldrich, USA) to remove the protecting group, thereby preparing a 5' peptide-PEG-double helix oligo RNA structure.

### Example 2-4: Preparation of 5' C₂₄-RNA structure

A RNA structure complementary to the RNA sequence of the 5' ligand-PEG-double stranded oligo RNA structure of Example 2-3 was synthesized by the RNA synthesis method of Example 2-1, and then treated with a C₂₄ tetradocosane reagent containing a disulfide bond according to a general RNA synthesis process to bond C₂₄ to the RNA structure by a phosphodiester bond, thereby synthesizing a 5' C₂₄-RNA structure (see FIG. 11).

### Example: 2-5: Recovery and annealing

Each of the single-stranded RNAs synthesized in Examples 2-1, 2-2, 2-3 and 2-4 was treated with 28% (v/v) ammonia in water bath at 60 **°C** to separate the synthesized RNAs and RNA structures from the CPGs, followed by deprotection. The deprotected, 5' ligand-PEG-double stranded RNA structures and 5' C₂₄-double stranded oligo RNA structures were treated with a 10:3:4 (v/v/v) mixture of N-methylpyrrolidone, triethylamine and triethylamine trihydrofluoride in an oven at 70 **°C** to remove 2'TBDMS (tert-butyldimethylsilyl).

The RNAs were separated from the reactants by high-performance liquid chromatography (HPLC) to separate the RNAs, and the molecular weights of the RNAs were measure by MALDI-TOF MS (SHIMADZU, Japan) to determine whether the RNAs were consistent with the desired nucleotide sequences and double-helix oligo RNA structures.

Next, in order to prepare a double-helix oligo RNA structure having a ligand bonded thereto, the ligand-bonded, PEG-RNA sense RNA and antisense RNA were mixed with each other in the same amount, and the mixture was added to 1X annealing buffer (30 mM HEPES, 100 mM Potassium acetate, 2 mM Magnesium acetate, pH 7.0∼7.5) and allowed to react in a constant-temperature water bath at 90 **°C** for 3 minutes, and then allowed to react at 37 **°C,** thereby preparing a desired double-helix oligo RNA structure having a ligand bonded to the 5' end of each of the strands. The prepared double-helix oligo RNA having the ligand bonded to the 5' end was subjected to electrophoresis to confirm the annealing of the strands.

### Example 3: Preparation of double-helix oligo RNA structure having ligand bonded to 3' end

Amine-CPG was synthesized into 3' amine-PEG-RNA using a PEG phosphoramidite regent, and then liked with NHS-ligand such as NHS-folate of Example 1-2 via an ester bond, thereby synthesizing a PEG-double stranded oligo RNA structure having a ligand bonded to the 3' end. The synthesized PEG-double stranded oligo RNA structure having a ligand bonded to the 3' end was linked with the hydrophobic material C₂₄ to form PEG-RNA-C₂₄ having a ligand bonded to the 3' end. The PEG-RNA-C₂₄ was annealed with a complementary RNA to synthesize a double-helix oligo RNA structure having a ligand bonded to the 3' end.

### Example 3-1: Preparation of 3' amine-PEG-RNA structure

Amine-CPG was treated with a polyethylene glycol phosphoramidite reagent according to a general RNA synthesis process to synthesize 3' CPG-amine-PEG. The 3' CPG-amine-PEG was synthesized into a 3' CPG-amine-PEG-RNA structure having a desired RNA sequence according to the RNA synthesis process of Example 2-1 (see FIG. 12).

### Example 3-2: Preparation of 3' amine-PEG RNA-C₂₄ structure

The 3' CPG-amine-PEG-RNA structure synthesized in Example 3-1 was treated with a C₂₄ tetradocosane reagent containing a disulfide bond according to a general RNA synthesis process to bond C₂₄ to the RNA via a phosphodiester bond, thereby synthesizing a 3' amine-PEG RNA-C₂₄ structure (see FIG. 13).

### Example 3-3: Preparation of PEG RNA-C₂₄ structure having ligand bonded to 3' end

The 3' amine-PEG-RNA-C₂₄ structure synthesized in Example 3-2 was treated with 28% ammonia in water bath at 60 **°C** to separate the synthesized 3' amine-PEG-double stranded oligo RNA structure and 3' amine-PEG-RNA-C₂₄ structure from the CPG, followed by deprotection. The deprotected 3' amine-PEG-RNA-C₂₄ structure treated with a 10:3:4 (v/v/v) mixture of N-methylpyrrolidone, triethylamine and triethylamine trihydrofluoride in an oven at 70 **°C** to remove 2' TBDMS (tert-butyldimethylsilyl). The separated 3' amine-PEG-RNA-C₂₄ structure was linked with a ligand material such as NHS-ligand by an ester bond, thereby synthesizing a PEG-RNA-C₂₄ structure having a ligand bonded to the 3' end.

### Example 3-3-1: Preparation of 3' folate-PEG-RNA structure

The amine-PEG-RNA-C₂₄ structure synthesized in Example 3-2 was linked with NHS-folate (synthesized in Example 1-2) by an ester bond to synthesize a 3' folate-PEG-RNA-C₂₄ structure (FIG. 14).

### Example 3-4: Preparation of complementary RNA structure

A single-stranded RNA complementary to the sequence of the 3' ligand-PEG-RNA-C₂₄ structure of Example 3-3 was synthesized to the RNA synthesis method of Example 2-1. The synthesized single-stranded RNAs were treated with 28% ammonia in water bath at 60 **°C** to separate the synthesized RNAs from the CPG, followed by deprotection. The deprotected RNAs were treated with a 10:3:4 (v/v/v) mixture of N-methylpyrrolidone, triethylamine and triethylamine trihydrofluoride in an oven at 70 **°C** to remove 2' TBDMS (2'tert-butyldimethylsilyl).

### Example 3-5: Annealing

The RNA and the 3' ligand-PEG RNA-C₂₄ structure reaction products were separated from the reactants by high-performance liquid chromatography (HPLC; LC-20A Prominence, SHIMADZU, Japan), and the molecular weights of the separated materials were measured by MALDI TOF-MS (SHIMADZU, Japan) to determine whether they were consistent with the desired nucleotide sequence and 3' ligand-PEG RNA-C₂₄ structure.

Next, in order to prepare a double-helix oligo RNA structure having a ligand bonded thereto, the ligand-bonded, PEG-RNA sense RNA and antisense RNA were mixed with each other in the same amount, and the mixture was added to 1X annealing buffer (30 mM HEPES, 100 mM Potassium acetate, 2 mM Magnesium acetate, pH 7.0∼7.5) and allowed to react in a constant-temperature water bath at 90 **°C** for 3 minutes, and then allowed to react at 37 **°C,** thereby preparing a desired double-helix oligo RNA structure having a ligand bonded to the 3' end of each of the strands. The prepared double-helix oligo RNA having the ligand bonded to the 3' end was subjected to electrophoresis to confirm the annealing of the strands.

### Example 4: Formation of nanoparticles composed of double-helix oligo RNA structures having ligand bonded thereto

The double-helix oligo RNA structures having the ligand to the 5' end, and the double-helix oligo RNA structures having the ligand to the 3' end, synthesized in Examples 2 and 3, form a nanoparticle (i.e., micelle) composed of ligand-bonded double-helix oligo RNA structures by hydrophobic interactions between the hydrophobic materials bonded to the ends of the double-helix oligo RNAs (see FIG. 1). Size and critical micelle concentration (CMC) measurements and transmission electron microscope (TEM) analysis for a nanoparticle composed of the 5' folate-ligand-double stranded oligo RNA synthesized in Example 2 were measured to confirm the formation of the nanoparticle.

### Example 4-1: Measurement of particle size of nanoparticle composed of 5' folate-double stranded oligo RNA structures

The size of the nanoparticle was measured by zeta-potential measurement. Specifically, the 5' folate-double stranded oligo RNA structures were dissolved in 1.5 **Mℓ** DPBS (Dulbecco's Phosphate Buffered Saline) at a concentration of 50 **µg/Mℓ,** and then homogenized with a sonicator (Wiseclean, DAIHAN, Korea) (700 W; amplitude: 20%). The size of the homogenized nanoparticles was measured with a Zetasizer (Nano-ZS, MALVERN, GB) under the following conditions: refractive index: 1.459, absorption index: 0.001, temperature of solvent PBS (phosphate buffered saline: 25 **°C,** viscosity at that temperature: 1.0200; and refractive index: 1.335. Each measurement consisted of 20 readings and was repeated three times.

It was found that nanoparticles (folate-SAMiRNA) composed of the folate-bonded double-helix oligo RNA structures had a size of about 100-200 nm. A lower polydisperse index (PDI) value indicates a more uniform distribution of the particles. The PDI value of folate-SAMiRNA was measured to be less than 0.4, suggesting that nanoparticles having a relatively uniform size were formed. It was found that the size of the nanoparticles composed of such structures is suitable for uptake into cells by endocytosis (Nanotoxicology: nanoparticles reconstruct lipids.Nat Nanotechnol. 2009 Feb;4(2):84-5) (see FIG. 15(A)).

### Example 4-2: Measurement of critical micelle concentration of nanoparticles composed of double-helix oligo RNA structures

An amphiphilic material containing both an oleophilic group and a hydrophilic group in the molecule can act as a surfactant. When a surfactant is dissolved in an aqueous solution, the hydrophobic moieties go inward in order to avoid contact with the water, and the hydrophilic moieties go outward, thereby forming a micelle. The concentration at which the micelle is first formed is defined as critical micelle concentration (CMC). A method of measuring the CMC using a fluorescent dye is based on a rapid change in the slope of the fluorescence intensity graph of a fluorescent dye before and after formation of the micelle.

For measurement of the critical micelle concentration of the nanoparticles composed of the folate-bonded double stranded oligo RNA structures, 0.04 mM DPH (1,6-Diphenyl-1,3,5-hexatriene, Sigma Aldrich, USA) as a fluorescent dye was prepared. 1 nmole/**µℓ** of the 5' folate-double stranded oligo RNAs synthesized in Example 2 was diluted with DPBS serially from 0.0977 **µg/Mℓ** to 50 **µg/Mℓ,** thereby preparing 180 **µℓ** of each of 5' folate-double stranded oligo RNA structure samples. To the prepared sample, 20 **µℓ** of each of 0.04 mM DPH in methanol and methanol alone as a control was added and well agitated. Then, homogenization using a sonicator (Wiseclean, DAIHAN, Korea) was performed in the same manner as described in Example 4-1 (700 W; amplitude: 20%). Each of the homogenized samples was allowed to react at room temperature under a light-shielded condition for about 24 hours, and the fluorescence intensities (excitation: 355 nm, emission: 428 nm, top read) were measured. Because the measured fluorescence intensities are used to determine the relative fluorescence intensity, the relative fluorescence intensity ([fluorescence intensity of DPH-containing sample]-[fluorescence intensity of sample containing methanol alone]) at the same concentration was calculated and graphically shown on the Y-axis as a function of the log value of the concentration of 5' folate-double stranded oligo RNA structures (X-axis) (see FIG. 15(B)).

The fluorescence intensities measured at various concentrations increase as the concentration increases, and the point at which the concentration increases rapidly is the CMC concentration. Thus, the low-concentration region in which the fluorescence did not increase and the high-concentration region in which the fluorescence intensity increased were divided into several points to draw trend lines, and the X-axis value at which the two trend lines crossed with each other was determined as the CMC concentration (FIG. 15 (B)). The measured CMC of the folate-double stranded oligo RNA structure was very low (1.33 **µg/Mℓ**), suggesting that Folate-SAMiRNA can easily form micelles at a very low concentration.

### Example 4-3: Observation of double-helix oligo RNA structure by transmission electron microscope (TEM)

The morphology of nanoparticles formed of the folate-double stranded oligo RNA structures was observed by a transmission electron microscope (TEM).

Specifically, the folate-double stranded oligo RNA structures were dissolved in DPBS (Dulbecco's Phosphate-Buffered Saline) to a final concentration of 100 ng/**Mℓ**, and then homogenized with a sonicator (Wiseclean, DAIHAN, Korea) (700 W; amplitude: 20%). The nanoparticles formed of the folate-double stranded oligo RNA structures were observed by negative staining with a material having high electron density. The nanoparticles observed by the transmission electron microscope (TEM) had a size similar to that of the nanoparticle size measured in Example 4-1, suggesting that the nanoparticles were easily formed.

### Example 5: In vitro delivery of ligand-bonded double stranded RNA structures

In order to examine whether nanoparticles (folate-SAMiRNA) composed of the 5' folate-double stranded oligo RNA structures synthesized in Example 2 show improved effects of the double-helix oligo RNA *in vitro,* the KB cell line that overexpresses the folate receptor was cultured in the presence or absence of folate without transfection. As a result, it was found that the bonded ligand enhanced the intracellular delivery efficiency of SAMiRNA and that SAMiRNA exhibited the effect of inhibiting the expression of the target gene.

### Example 5-1: Culture of tumor cell line

The human oral epithelial carcinoma cell line (KB) purchased from the American type Culture Collection (ATCC) was cultured in folate-free RPMI-1640 medium (Gibco, USA) supplemented with 10 %(v/v) FBS, 100 units/**Mℓ** penicillin and 100 **µg/Mℓ** streptomycin, under the conditions of 37 **°C** and 5 % (v/v) CO₂.

### Example 5-2: Transfection of tumor cell line with folate-SAMiRNA

The tumor cells (1.3 × 10⁵ cells/well) cultured in Example 5-1 were cultured in a folate-free RPMI-1640 medium in a 6-well plate for 18 hours under the conditions described in Example 5-1, and then the medium was removed and the same amount of Opti-MEM medium was added to each well.

Nanoparticles (SAMiRNA-Sur) composed of double-helix oligo RNA structures comprising a sequence of SEQ ID NO: 1 that inhibits the expression of the target gene survivin, nanoparticles (SAMiRNA-Con) composed of double-helix oligo RNA-polymer structures comprising a control sequence of SEQ ID NO: 2, and nanoparticles (Folate-SAMiRNA-Sur) composed of folate ligand-bonded double stranded oligo RNA structures comprising a sequence of SEQ ID NO: 1, were dissolved in DPBS at a concentration of 50 **µg/Mℓ** according to the same method as described in Example 4-1, and were homogenized by sonication, thereby obtaining homogenized nanoparticles composed of each of the structures.

In order to form a condition in which an excessive amount of folate in Opti-MEM medium, folate was additionally added to the medium to form a condition containing 1 mM folate and a condition to which folate was not additionally added. Then, cells were treated with 200 nM of each of the samples and cultured under the conditions of 37 **°C** and 5 %(v/v) CO₂ for 48 hours.

### Example 5-3: Relative quantitative analysis of mRNA of survivin gene

Total RNA was extracted from the transfected cell line of Example 5-2 and synthesized into cDNA, and then the relative expression level of survivin was quantified by real-time PCR according to the method described in Korean Patent Laid-Open Publication No. 2009-0042297

SAMiRNA-Con is a test group treated with nanoparticles composed of double-helix oligo RNA structures comprising a control sequence of SEQ ID NO: 2, and SAMiRNA-Sur is a test group treated with nanoparticles composed of double- helix oligo RNA structures comprising a sequence of SEQ ID NO: 1 (survivin sequence). Folate-SAMiRNA-Sur is a test group treated with nanoparticles composed of folate-double stranded oligo RNA structures comprising a sequence of SEQ ID NO: 1 (survivin sequence).

The degree of inhibition of the target mRNA expression was the expression level of the target gene in the test group treated with each of SAMiRNA-Sur and Folate-SAMiRNA-Sur relative to the expression level of the target gene in the test group treated with SAMiRNA-Con and was determined by comparative quantitation (see FIG. 16).

When an excessive amount of folate was present in the medium, it could be seen that the folate receptor in the KB cell line was saturated with an excessive amount of folate, and thus the effect of promoting intracellular internalization by the folate ligand bonded to SAMiRNA was masked, suggesting that the folate ligand influences the intracellular delivery efficiency of SAMiRNA to play a crucial role on the inhibition of mRNA expression of the target gene.

In the case of the group treated with SAMiRNA-Sur, there was no significant difference in the inhibition of expression of the target gene between the presence and absence of folate, but in the case of the group treated with folate-SAMiRNA-Sur, the inhibition of expression of the target gene was about two times higher in the absence of folate than in the presence of folate. In other words, when an excessive amount of folate was present, the change in the target gene expression inhibitory effect by the bonded folate ligand was not observed, but when folate was absent, the increase in the target gene expression inhibitory effect by the bonded folate ligand was observed.

Thus, it can be seen that nanoparticles composed of the ligand-bonded double-helix oligo RNA structures show enhanced intracellular delivery efficiency and increased inhibition of the target gene in cells in which the ligand receptor is overexpressed.

### Example 6: In vivo delivery of ligand-bonded double-helix oligo RNA structure

In order to examine whether nanoparticles (folate-SAMiRNA) composed of the 5' folate-double stranded oligo RNA structures synthesized in Example 2 enhance the effect of the double stranded oligo RNA under *in vivo* conditions, the nanoparticles were administered to a mouse having a tumor composed of the KB cell line overexpressing the folate receptor, and the effects of the delivered folate-SAMiRNA and SAMiRNA on the inhibition of expression of the target gene in the tumor tissue were examined.

### Example 6-1: Preparation of KB xenograft model

The KB cell line cultured in Example 5-1 was injected subcutaneously into each of 5-week-old nude mice (BALB/C nu) at a density of 1 X 10⁶ cells. After injection, the growth of the tumor was observed by measuring the lengths of the long axis and short axis of the tumor at 2-day intervals, and it was shown that the tumor grew to a volume of about 150-200 mm³ at 2 weeks after injection.

### Example 6-2: Ligand-bonded double-helix oligo RNA structures and administration of ligand-bonded double-helix oligo RNA structures

For administration into the KB xenograft model prepared in Example 6-1, the 5' folate-double stranded oligo RNA structures comprising the sequence of SEQ ID NO: 1, synthesized in Example 2, and double-stranded RNA structures having no ligand bonded thereto, were homogenized in the same manner as described in Example 4-1, thereby obtaining homogenized nanoparticles composed of double-helix oligo RNA structures. The homogenized nanoparticles were administered once into the tail vein of the KB xenograft models (n=4) at a dose of 5 mg/kg body weight), and the tumor tissue was collected at 48 or 72 hours after administration. Total RNA was extracted from the collected tumor tissue and synthesized into cDNA, and then the relative expression level of the survivin mRNA was quantified by real-time PCR according to the method described in Korean Patent Laid-Open Publication No. 2009-0042297 (see FIG. 17).

In FIG. 17, PBS is a test group administered with a solvent alone as a negative control, SAMiRNA is a test group administered with nanoparticles composed of the double-helix oligo RNA structures having no ligand bonded thereto, and Folate-SAMiRNA is a test group administered with nanoparticles composed of the 5' folate-double stranded oligo RNA structures.

The target gene expression inhibitory effect of SAMiRNA was higher at 72 hours after administration than at 48 hours after administration. The inhibition of expression of the target gene in the group administered with the ligand-bonded structure (Folate-SAMiRNA) was 160% at 48 hours after administration and 120% at 72 hours after administration.

Thus, it can be seen that the double-stranded RNA structure having the folate ligand bonded thereto is quickly delivered into the *in vivo* target tumor tissue that overexpresses the folate receptor, so that the effect of the double-helix oligo RNA is improved, and that the effect is maintained even with the passage of time.

### Example 7: Preparation of ASO-polymer conjugate

In the examples of the present invention, a survivin ASO was used in order to inhibit surviving (Biol. Proced. Online 2004; 6(1): 250-256). Survivin is a protein that is expressed commonly in most tumors or mutant cell lines tested to date and is expected to be an important target in anticancer therapy (Abbrosini G. et al. Nat. Med. 3(8):917-921,1997).

The ASOs used in the following examples are a survivin-specific sequence set forth in SEQ ID NO: 3 and a control sequence set forth in SEQ ID NO: 4.
(SEQ ID NO: 3) survivin ASO (ISIS 23722), 5-TGTGCTATTCTGTGAATT-3
(SEQ ID NO: 4) scrambled control (ISIS 28598), 5-TAAGCTGTTCTATGTGTT-3

The ASO sequences were synthesized by linking nucleotides by the phosphodiester bonds of the DNA backbone using β-cyanoethylphosphoramidite (Shina et al. NucleicAcidsResearch,12:4539-4557,1984).

In the ASO synthesis process, a cycle consisting of deblocking, coupling, capping and oxidation was repeated on a solid support (CPG) having a nucleoside attached thereto, thereby obtaining a desired DNA sequence

Specifically, in a deblocking step that is the first step, a CPG having a nucleoside attached thereto is treated with 3% trichloroacteic acid (TCA) to remove DMT (4,4'-dimethoxytrityl). In a coupling step that is the next step, nucleotide chains are linked to each other by the binding reaction between the 5'- hydroxyl group formed on the CPG in the previous step and a nucleoside phosphoramidite monomer having a desired sequence In a capping step that is the third step, a 5'-hydroxyl group that was not reacted in the coupling step is blocked in order to eliminate the formation of a nucleotide chain having an undesired nucleotide sequence in the coupling step of the next cycle. In the capping step, the unreacted 5'-hydroxyl group is acetylated by treating it with acetic anhydride and *N*-methylimidazole In an oxidation step that is the final step, the phosphitetriester bond between a 5'-hydroxyl group and phosphoramidite, formed in the coupling step, is converted into a phosphodiester bond. In this oxidation step, the phosphitetriester bond is treated with 0.02 M oxidizing solution (0.02 M-I2 in THF/pyridine/H₂O) to convert phosphate into phosphate. A series of processes for synthesizing the ASO were performed using a DNA synthesizer (384 Synthesizer, BIONEER, Korea).

In a process of synthesizing an ASO-polymer conjugate (3'PEG-ASO-5'lipid), an ASO was synthesized by deblocking, coupling, capping and oxidation from a 3' PEG-CPG support having the hydrophilic material PEG at the 3' end, and the C₂₄ hydrophobic material tetradocosane containing a disulfide bond was attached to the 5' end, thereby preparing a desired ASO-polymer conjugate (see Korean Patent Laid-Open Publication No. 2009-0042297).

Also, in order to prepare a 3'ligand-PEG-ASO-5'lipid, PEG was attached to a CPG having a functional group such as an amine group attached thereto, using a PEG phosphoramidite reagent by a process consisting of deblocking, coupling, capping and oxidation, and the C₂₄ hydrophobic material tetradocosane containing a disulfide bond was attached to the 5' end, thereby preparing a 3'-functional group-PEG-ASO-5'-lipid to which a desired ligand can be attached. After the completion of synthesis, the reaction product was treated with 28% (v/v) ammonia in water bath at 60 **°C** to separate the ASO-polymer conjugate, to which a ligand can be attached, from the CPG. Then, a ligand was attached to the conjugate by the functional group, thereby preparing an ASO-polymer conjugate (3' ligand-PEG-ASO-5'lipid).

Meanwhile, in order to synthesize an ASO-polymer conjugate (3'lipid-ASO-5'PEG), an ASO was synthesized on a CPG having a functional group such as an amine group by a process consisting of deblocking, coupling, capping and oxidation, and PEG was attached to the 5' end using PEG phosphoramidite, thereby preparing a functional group-ASO-hydrophilic polymer conjugate. After completion of synthesis of the functional group-ASO-hydrophilic polymer conjugate, the reaction product was treated with 28%(v/v) ammonia in water bath at 60 **°C** to separate the functional group-ASO-hydrophilic polymer conjugate from the CPG, and then a hydrophobic material was attached to the conjugate via the functional group, thereby preparing an ASO-polymer conjugate having desired hydrophilic and hydrophobic materials attached thereto. Then, the ASO-polymer conjugate was separated and purified from the reactants by high-performance liquid chromatography (HPLC) (LC-20A Prominence, SHIMADZU, Japan), and the molecular weights of the ASO and the ASO-polymer conjugate were measured by MALDI TOF-MS (SHIMADZU, Japan)to determine whether the nucleotide sequence to be synthesized was obtained.

### Example 8: Synthesis of ASO-polymer conjugate modified with phosphothioate

The ASO used in this Example was obtained by substituting the phosphate group of the DNA backbone with a phosphothioate group to obtain S-oligos and linking the S-oligos by phosphothioate bonds.

Specifically, an ASO comprising S-oligos was synthesized by a process consisting of deblocking, coupling, capping and oxidation, in which the oxidation step was performed by treatment with 0.1 M sulfurizing reagent in place of 0.02 M oxidizing solution. By this ASO synthesis process, phosphothioate-modified ASOs having sequences of SEQ ID NOS: 3 and 4 were synthesized (Shina etal.NucleicAcidsResearch,12:4539-4557,1984). The remaining synthesis processes used in this Example were similar to those used in Example 7.

In order to synthesize an ASO in which 4 nucleotides at both ends (5' and 3' ends) were modified with 2-OCH₃ (methoxy), a nucleoside in the region modified in the form of 2'-OCH₃-DNA was synthesized using 2'-OCH₃-DNA-cyanoethyl phosphoramidite [rA(Bz),rC(Ac),rG(ibu),rU] to substitute the DNA backbone with phosphothiolate, and then, as described above, the DNA backbone was synthesized by linking S-oligos via phosphothiolate bonds.

In order to prepare a phosphothiolate-modified ASO-polymer conjugate, an ASO conjugate modified with phosphothiolate was synthesized on a 3'PEG-CPG support by a cycle consisting of deblocking, coupling, capping and oxidation as known in the art (see Korean Patent Laid-Open Publication No. 2009-0042297), and then the C₂₄ hydrophobic material tetradocosane containing a disulfide bone was attached to the 5' end, thereby preparing a desired phosphothiolate-modified ASO-polymer conjugate.

Also, in order to attach a ligand to the end of the hydrophilic material of the phosphothiolate-modified ASO-polymer conjugate, a functional group to which a ligand can be attached was attached to the 3' CPG, and then a hydrophilic material was bonded to the CPG, and the above-described reaction was performed, thereby preparing an ASO-polymer conjugate to which a ligand can be attached.

After the completion of synthesis of the ASO-polymer conjugate, the reaction product was treated with 28%(v/v) ammonia in water bath at 60 **°C** to separate the ASO and the ASO-polymer conjugate from the CPG. Then, the ASO-polymer conjugate was separated and purified from the reactants by high-performance liquid chromatography (HPLC) (LC-20A Prominence, SHIMADZU, Japan), and the molecular weights of the ASO and the ASO-polymer conjugate were measured by MALDI TOF-MS (SHIMADZU, Japan) to determine whether the nucleotide sequence to be synthesized was obtained (see FIG. 19).

### Example 9: Evaluation of stability of ASO-polymer conjugate under conditions mimicking in vivo conditions

In order to examine whether the stability of the ASO-polymer conjugates, synthesized and separated in Examples 7 and 8, was increased compared to that of the original ASO having no polymer attached thereto, the following experiment was performed. Specifically, an ASO having no polymer attached thereto and the ASO-polymer conjugate were incubated in 30 and 50%(v/v) FBS (fetal bovine serum)-containing media, which mimic *in vivo* conditions, for 0, 3, 5, 7 and 10 days, and then the degradation of the ASO-polymer conjugate was analyzed comparatively with that of the original ASO by electrophoresis or polymerase chain reaction (PCR).

As a result, the ASO-polymer conjugate was stable regardless of the concentration of FBS, even though PEG was separated from the ASO with the passage of time, whereas the stability of the ASO having no polymer attached thereto started to decrease after day 3.

### Example 10: Analysis of physical properties of nanoparticle composed of ASO-polymer conjugates

The ASO-polymer conjugates form a nanoparticle composed of the ASO-polymer conjugates by interaction between the hydrophobic materials attached to the ends of the ASOs (see FIG. 18). The size and critical micelle concentration (CMC) of the nanoparticles composed of the ASO-polymer conjugates were measured by zeta-potential measurement.

### Example 10-1: Measurement of nanoparticles composed of ASO-polymer conjugates

The size of the nanoparticles composed of the ASO-polymer conjugates, prepared in Example 8 and comprising a sequence of SEQ ID NO: 3, was measured by zeta potential measurement. Specifically, 50 **µg** of the ASO-polymer conjugates were dissolved in 1 ml of DPBS (Dulbecco's Phosphate Buffered Saline), and then homogenized with a sonicator (Wiseclean, DAIHAN, Korea) (700 W; amplitude: 20%). The size of the homogenized nanoparticles was measured by zeta potential measurement device (Nano-ZS, MALVERN, England) under the following conditions: refractive index: 1.454, absorption index: 0.001, temperature of water as a solvent: 25 **°C.** Each measurement consisted of 20 size readings and was repeated three times.

It could be observed that the nanoparticles formed of the ASO-polymer conjugates had a size of 100-200 nm and a polydispersity index (PDI) of less than 0.4 (see FIG. 20(A)). A lower polydisperse index (PDI) value indicates a more uniform distribution of the particles. Thus, it can be seen that nanoparticles formed of the ASO-polymer conjugates have a relatively uniform size, which is suitable for uptake into cells by endocytosis (Kenneth A. Dawson et al. nature nanotechnology 4:84-85, 2009).

### Example 10-2: Measurement of critical micelle concentration of ASO-polymer conjugates

An amphiphilic material containing both an oleophilic group and a hydrophilic group in the molecule can act as a surfactant. When a surfactant is dissolved in an aqueous solution, the hydrophobic moieties go inward in order to avoid contact with the water, and the hydrophilic moieties go outward, thereby forming a micelle. The concentration at which the micelle is first formed is defined as critical micelle concentration (CMC). A method of measuring the CMC using a fluorescent dye is based on a rapid change in the slope of the fluorescence intensity graph of a fluorescent dye changes rapidly before and after formation of the micelle.

For measurement of the critical micelle concentration of the nanoparticles composed of the ASO-polymer conjugates, 0.04 mM DPH (1,6-Diphenyl-1,3,5-hexatriene, SIGMA, USA) as a fluorescent dye was prepared. 1 nmole/**µℓ** of the ASO-polymer conjugates comprising a sequence of SEQ ID NO: 3 were diluted with DPBS serially from 0.0977 **µg/Mℓ** to 50 **µg/Mℓ,** thereby preparing 180 **µℓ** of each of ASO-polymer conjugate samples. To the prepared sample, 20 **µℓ** of each of 0.04 mM DPH in methanol and methanol alone as a control was added and well agitated. Then, homogenization using a sonicator (Wiseclean, DAIHAN, Korea) was performed in the same manner as described in Example 10-1 (700 W; amplitude: 20%). Each of the homogenized samples was allowed to react at room temperature under a light-shielded condition for about 24 hours, and the fluorescence intensities (excitation: 355 nm, emission: 428 nm, top read) were measured. Because the measured fluorescence intensities are used to determine the relative fluorescence intensity, the relative fluorescence intensity ([fluorescence intensity of DPH-containing sample]-[fluorescence intensity of sample containing methanol alone]) at the same concentration was calculated and graphically shown on the Y-axis as a function of the log value of the concentration of ASO-polymer conjugates (X-axis) (see FIG. 20(B)).

The fluorescence intensities measured at various concentrations increase as the concentration increases, and the point at which the concentration increases rapidly is the CMC concentration. Thus, the low-concentration region in which the fluorescence did not increase and the high-concentration region in which the fluorescence intensity increased were divided into several points to draw trend lines, and the X-axis value at which the two trend lines crossed with each other was determined as the CMC concentration. The measured CMC of the ASO-polymer conjugates was very low (1.56 **µg/Mℓ**), suggesting that nanoparticles formed of the ASO-polymer conjugates can easily form micelles even at a very low concentration.

### Example 11: Inhibition of expression of target gene in tumor cell line by ASO-polymer conjugate and transfection reagent

Each of the ASO having no polymer conjugate thereto, and the ASO-polymer conjugate, prepared in Example 8, was transfected into a human colorectal cancer cell line (SW480) as a tumor cell line, and the expression patterns of survivin in the transfected tumor cell line were analyzed.

### Example 11-1: Culture of tumor cell line

The human colorectal cancer cell line (SW480) obtained from the American type Culture Collection (ATCC) was cultured in a growth medium (Leibovitz's L-15 medium, GIBCO/Invitorgen; USA), supplemented with 10 % (v/v) FBS, 100 units/ml penicillin and 100 **µg**/ml streptomycin, under the conditions of 37 **°C** and 5% (v/v) carbon dioxide (CO₂).

### Example 11-2: Transfection of tumor cell line with ASO-polymer conjugate

Each of the ASO having no polymer conjugate thereto, and the ASO-polymer conjugate, prepared in Example 8, was transfected into a human colorectal cancer cell line (SW480) as a tumor cell line, and the expression patterns of survivin in the transfected tumor cell line were analyzed.

The tumor cell line cultured in Example 11-1 was cultured in a growth medium (Leibovitzs L-15 medium, GIBCO/Invitorgen; USA) in a 6-well plate at a density of 1.3 × 10⁵ cells for 18 hours under the conditions described in Example 11-1, and then the medium was removed, and 800 **µℓ** of Opti-MEM (modification of Eagle's Minimum Essential Media, GIBCO/Invitorgen; USA) medium was added to each well.

Meanwhile, 2 **µℓ** of Lipofectamine™ 2000 and 198 **µℓ** of Opti-MEM medium were mixed with each other and allowed to react at room temperature for 5 minutes. The reaction product was treated with 25 pmole/**µℓ** of each of the ASO-polymer conjugates, prepared in Examples 7 and 8, to final concentrations of 10, 50 and 100 nM, and was then allowed to react at room temperature for 20 minutes, thereby preparing transfection solutions.

Next, 200 **µℓ** of each of the transfection solutions was added to each well containing the tumor cell line and OptiMEM, and then the cells were cultured for 6 hours, followed by removal of the Opti-MEM medium Next, 2.5 Mℓ of growth medium (Leibovitz's L-15 medium, GIBCO/Invitorgen; USA) was added to each well, and then the cells were cultured for 24 hours under the conditions of 37 °C and 5 %(v/v) carbon dioxide (CO₂).

### Example 11-3: Relative quantitative analysis of mRNA of survivin gene

Total RNA was extracted from the transfected cell line of Example 11-2 and synthesized into cDNA, and then the mRNA level of the survivin gene was comparatively quantified by real-time PCR according to the method described in Korean Patent Laid-Open Publication No. 2009-0042297 (see FIG. 21)

To analyze the target gene expression inhibitory effects of the ASO having no polymer conjugated thereto and the ASO-polymer conjugate, cells were transfected with each of the ASO and the ASO-polymer conjugate together with the transfection reagent, and then the mRNA expression levels of the survivin gene in the cells were analyzed. As a result, it was found that the inhibition of expression of the survivin gene by the ASO-polymer conjugate was similar to that by the ASO having no polymer conjugated thereto, suggesting that the conjugated polymer does not interfere with the mechanism of action of the ASO.

### Example 12: Inhibition of expression of target gene in tumor cell line by ASO-polymer conjugate alone

Each of the ASO-polymer conjugates prepared in Examples 7 and 8 was transfected into a human colorectal cancer cell line (SW480) as a tumor cell line, and the expression patterns of survivin in the transfected tumor cell line were analyzed.

### Example 12-1: Culture of tumor cell line

The human colorectal cancer cell line (SW480) obtained from the American type Culture Collection (ATCC) was cultured in a growth medium (Leibovitz's L-15 medium, GIBCO/Invitorgen; USA), supplemented with 10 %(v/v) FBS, 100 units/ml penicillin and 100 µg/ml streptomycin, under the conditions of 37 °C and 5%(v/v) carbon dioxide (CO₂).

### Example 12-2: Transfection of tumor cell line with ASO-polymer conjugate

The tumor cell line cultured in Example 12-1 was cultured in a growth medium (Leibovitz's L-15 medium, GIBCO/Invitorgen; USA)in a 6-well plate at a density of 1.3 × 10⁵ cells for 18 hours under the conditions described in Example 5-1, and then the medium was removed, and 800 µℓ of Opti-MEM medium was added to each well.

100 µℓ of Opti-MEM medium and 5, 10 or 100 µℓ (500 nM, 1 µM or 10 µM) of each of the ASO-polymer conjugates (1 nmole/µℓ) prepared in Examples 1 and 2 were mixed with each other and homogenized with a sonicator (Wiseclean, DAIHAN, Korea) in the same manner as described in Example 4-1 (700 W; amplitude: 20 %), thereby preparing transfection solutions containing homogenized nanoparticles formed of the ASO-hydrophobic material conjugates.

Next, 100 µℓ of each of the transfection solutions was added to each well containing the tumor cell line and Opti-MEM, and the cells were cultured for 24 hours, after which 1 ml of 20% FBS-containing growth medium (Leibovitz's L-15 medium, GIBCO/Invitorgen; USA) was added thereto. Then, the cells were additionally cultured for 24 hours under the conditions of 37 °C and 5% (v/v) carbon dioxide (CO₂). Thus, the cells were cultured for a total of 48 hours after treatment with the ASO-polymer conjugate.

### Example 12-3: Relative quantitative analysis of mRNA of survivin gene

Total RNA was extracted from the transfected cell line of Example 12-2 and synthesized into cDNA, and then the mRNA levels of the survivin gene were comparatively quantified by real-time PCR according to the method described in Korean Patent Laid-Open Publication No. 2009-0042297

The inhibition of mRNA expression of the survivin gene was compared between the ASO having no polymer conjugated thereto and the ASO-polymer conjugate under a condition containing no transfection reagent. As a result, it could be seen that the ASO-polymer inhibited the expression of the target gene at a relatively low concentration compared to the non-conjugated ASO under a condition containing no transfection reagent.

Thus, it can be seen that the ASO-polymer conjugates synthesized in the present invention or nanoparticles composed of the ASO-polymer conjugates are delivered into cells even under a condition containing no transfection reagent so that the ASO inhibits the expression of the target gene.

### INDUSTRIAL APPLICABILITY

As described above, the novel oligonucleotide structure according to the present invention and a pharmaceutical composition comprising the same can be used for the treatment of cancer and infectious diseases in a very efficient and useful manner.

Although the present invention has been described in detail with reference to the specific features, it will be apparent to those skilled in the art that this description is only for a preferred embodiment and does not limit the scope of the present invention. Thus, the substantial scope of the present invention will be defined by the appended claims and equivalents thereof.

## Claims

1. An antisense oligonucleotide (ASO)-polymer conjugate represented by the following formula 5:
Formula 5 A-X-R-Y-B
wherein one of A and B is a hydrophilic material, the other one is a hydrophobic material, X and Y are each a simple covalent bond or a linker-mediated covalent bond independently of each other, and R is an ASO.

2. The antisense oligonucleotide (ASO)-polymer conjugate of claim 1, wherein the ASO is composed of 10-50 oligonucleotides.

3. The antisense oligonucleotide (ASO)-polymer conjugate of claim 2, wherein the oligonucleotides
comprises modification comprising the substitution of an -OH group at the 2' carbon position of the sugar moiety of one or more nucleotides with -CH₃ (methyl), -OCH₃, -NH₂, -F (fluorine), -O-2-methoxyethyl, -O-propyl, -O-2-methylthioethyl, -0-3-aminopropyl, -O-3-dimethylaminopropyl, -O-*N*-methylacetamido or -O-dimethylamidoxyethyl; the substitution of oxygen in the sugar moiety of the nucleotide with sulfur; modification of the bond between the nucleotides into one or a combination of two or more selected from the group consisting of a phosphorothioate, boranophosphophate and methyl phosphonate bond, or is modified in the form of PNA (peptide nucleic acid) or LNA (locked nucleic acid).

4. The antisense oligonucleotide (ASO)-polymer conjugate of claim 1, wherein the hydrophilic material has a molecular weight of 200-10,000.

5. The antisense oligonucleotide (ASO)-polymer conjugate of claim 4, wherein the hydrophilic material is selected from the group consisting of polyethylene glycol, polyvinyl pyrolidone, and polyoxazoline.

6. The antisense oligonucleotide (ASO)-polymer conjugate of claim 1, wherein the hydrophobic material has a molecular weight of 250-1,000.

7. The antisense oligonucleotide (ASO)-polymer conjugate of claim 6, wherein the hydrophobic material is either a C₁₂-C₅₀ hydrocarbon or cholesterol.

8. The antisense oligonucleotide (ASO)-polymer conjugate of claim 1, wherein the covalent bond is either a non-degradable bond or a degradable bond.

9. The antisense oligonucleotide (ASO)-polymer conjugate of claim 8, wherein the non-degradable bonds is either an amide bond or a phosphate bone or wherein the degradable bonds is selected from the group consisting of a disulfide bond, an acid-degradable bond, an ester bond, an hydride bond, a biodegradable bond or an enzymatically degradable bond.

10. An ASO-polymer conjugate comprising a ligand bonded to a hydrophilic material of the ASO-polymer conjugate of any one of claims 1 to 9.

11. A method for preparing an ASO-polymer conjugate, the method comprising the steps of:
(a) covalently bonding a hydrophilic material to a solid support;
(b) synthesizing an ASO on the solid support comprising the hydrophilic material;
(c) covalently bonding a hydrophobic material to the 5' end of the ASO on the solid support; and
(d) separating and purifying the resulting ASO-polymer conjugate from the solid support.

12. A method for preparing an ASO-polymer conjugate, the method comprising the steps of:
(a) synthesizing an ASO on a solid support having a functional group bonded thereto;
(b) covalently bonding a hydrophilic material to the 5' end of the ASO;
(c) separating the hydrophilic material-bonded ASO conjugate from the solid support; and
(d) covalently bonding a hydrophobic material to the 3' end of the ASO separated from the solid support.

13. A method for preparing an ASO-polymer conjugate comprising a ligand attached thereto, the method comprising the steps of:
(a) bonding a hydrophilic material to a solid support having a functional group attached thereto;
(b) synthesizing an ASO on the solid support having the functional group-hydrophilic material bonded thereto;
(c) covalently bonding a hydrophobic material to the 5' end of the ASO;
(d) separating an ASO-polymer conjugate, obtained in step (c), from the solid support; and
(e) bonding a ligand to the hydrophilic material of the ASO-polymer conjugate separated from the solid support.

14. A method for preparing an ASO-polymer conjugate comprising a ligand attached thereto, the method comprising the steps of:
(a) synthesizing an ASO on a solid support having a functional group attached thereto;
(b) covalently bonding a hydrophilic material to the end of the ASO;
(c) covalently bonding a ligand to the ASO-hydrophilic material conjugate;
(d) separating an ASO-hydrophilic polymer-ligand conjugate, which has the functional group attached thereto, from the solid support; and
(e) covalently bonding a hydrophobic material to the 3' end of the ASO of the conjugate separated from the solid conjugate.

15. A nanoparticle comprising the ASO-polymer conjugate of any one of claims 1 to 10.

16. A pharmaceutical composition comprising a pharmaceutically effective amount of the ASO-polymer conjugate of any one of claims 1 to10 or the nanoparticle of claim 15.


<110>    BIONEER CORPORATION

<120>    NOVEL OLIGONUCLEOTIDE CONJUGATES AND USE THEREOF

<130>    27200EP1

<140>    20 172 761.7

<141>    2012-12-14

<150>    KR 10-2011-0135162
<151>    2011-12-15

<150>    KR 10-2012-0001710
<151>    2012-01-05

<160>    4

<170>    KoPatentIn 3.0

<210>    1
<211>    19
<212>    RNA
<213>    Artificial Sequence

<220>
<223>    survivin sequence for SAMiRNA-Sur


<400>    1
aaggagauca acauuuuca                                                    19


<210>    2
<211>    19
<212>    RNA
<213>    Artificial Sequence

<220>
<223>    control sequence for SAMiRNA-Con


<400>    2
cuuacgcuga guacuucga                                                    19


<210>    3
<211>    18
<212>    DNA
<213>    Artificial Sequence

<220>
<223>    survivin ASO


<400>    3
tgtgctattc tgtgaatt                                                     18


<210>    4
<211>    18
<212>    DNA
<213>    Artificial Sequence

<220>
<223>    scrambled control

<400>    4
taagctgttc tatgtgtt                                                    18